# EUROPÄISCHE PATENTSCHRIFT

(11) **EP 1 517 914 B1**
(45) Veröffentlichungstag und Bekanntmachung des Hinweises auf die Patenterteilung: **31.08.2005**
(21) Anmeldenummer: 03757065.2
(22) Anmeldetag: 11.06.2003
(51) Int. Cl.: C07J 1/00

(54) **9-ALPHA-SUBSTITUIERTE ESTRATRIENE ALS SELEKTIV WIRKSAME ESTROGENE**
9-ALPHA-SUBSTITUTED ESTRATRIENES AS SELECTIVELY ACTIVE ESTROGEN
ESTRATRIENES 9-ALPHA SUBSTITUES SERVANT D'OESTROGENES SELECTIVEMENT ACTIFS

(30) Priorität: 11.06.2002 DE 10226326
(43) Veröffentlichungstag der Anmeldung: 30.03.2005
(73) Patentinhaber: Schering Aktiengesellschaft, 13353 Berlin (DE)
(72) Erfinder: KOSEMUND, Dirk, 99091 Erfurt (DE); MÜLLER, Gerd, 07745 Jena (DE); HILLISCH, Alexander, 07743 Jena (DE); FRITZEMEIER, Karl-Heinrich, 15503 Berlin (DE); MUHN, Peter, 13465 Berlin (DE)
(86) Internationale Anmeldenummer: PCT/EP2003/006172
(87) Internationale Veröffentlichungsnummer: WO 2003/104253

(56) Entgegenhaltungen:
- DE-A- 19 906 159
- US-A- 3 960 841

## Beschreibung

### Feld der Erfindung

Die vorliegende Erfindung bezieht sich auf neue Verbindungen als pharmazeutische Wirkstoffe, die in vitro eine höhere Affinität an Estrogenrezeptorpräparationen von Rattenprostata als an Estrogenrezeptorpräparationen von Rattenuterus und in vivo eine präferentielle Wirkung im Ovar im Vergleich zum Uterus aufweisen, deren Herstellung, ihre therapeutische Anwendung und pharmazeutischen Darreichungsformen, die die neuen Verbindungen enthalten.
Bei den chemischen Verbindungen handelt es sich um neue steroidale gewebeselektive Estrogene.

### Hintergrund der Erfindung

Die Effizienz von Estrogenen in der Behandlung von hormondefizienzbedingten Symptomen wie Hitzewallungen, Atrophie von Estrogenzielorganen und Inkontinenz, sowie die erfolgreiche Anwendung von Estrogen-Therapien zur Verhinderung von Knochenmasseverlust bei peri- und postmenopausalen Frauen, ist gut belegt und allgemein akzeptiert (Grady et al.1992, Ann Intern Med 117: 1016-1037). Ebenso ist gut dokumentiert, dass die Estrogenersatztherapie bei postmenopausalen Frauen oder bei Frauen mit anders bedingter ovarieller Dysfunktion, das Risiko von Herzkreislauferkrankungen gegenüber nicht estrogenbehandelten Frauen reduziert (Grady et al., loc. cit.).

In der herkömmlichen Estrogen- oder Hormonersatztherapie (Hormone Replacement Therapy = HRT) werden natürliche Estrogene, wie Estradiol und konjugierte Estrogene aus Pferdeurin entweder allein oder in Kombination mit einem Gestagen eingesetzt. Anstelle der natürlichen Estrogene können auch durch Veresterung erhaltene Derivate, - wie - z.B. das 17β-Estradiol-valerat, eingesetzt werden.
Wegen der stimulierenden Wirkung der verwendeten Estrogene auf das Endometrium, die zu einer Erhöhung des Endometriumkarzinomrisikos führt (Harlap S 1992. Am J Obstet Gynecol 166: 1986-1992), werden in der Hormonersatztherapie vorzugsweise Estrogen/Gestagen-Kombinationspräparate eingesetzt, Die gestagene Komponente in der Estrogen/Gestagen-Kombination vermeidet eine Hypertrophie des Endometriums, allerdings ist mit der gestagen-haltigen Kombination auch das Auftreten ungewünschter Zwischenblutungen verknüpft.

Eine neuere Alternative zu den Estrogen/Gestagen-Kombinationspräparaten stellen selektive Estrogene dar. Bisher werden unter selektiven Estrogenen solche Verbindungen verstanden, die estrogenartig auf Gehirn, Knochen und Gefäßsystem, aufgrund ihrer antiuterotrophen (d.h. antiestrogenen) Partialwirkung aber nicht proliferativ auf das Endometrium wirken.

Eine Klasse von Substanzen, die das gewünschte Profil eines selektiven Estrogens teilweise erfüllen, sind die sogenannten, Selective Estrogen Receptor Modulators' (SERM) (R.F. Kauffman, H.U. Bryant 1995, DNAP B (9): 531-539). Es handelt sich hierbei um Partialagonisten des Estrogenrezeptorsubtyps, ERα'. Dieser Typ von Substanzen ist allerdings ineffektiv hinslchtlich der Therapie akuter postmenopausaler Beschwerden, wie z.B: Hitzewallungen. Als Beispiel für ein SERM sel das kürzlich für die Indikation Osteoporose eingeführte Raloxifen genannt.

DE-A-19906159 beschreibt neue Verbindungen als pharmazeutische Wirkstoffe, die in vitro eine höhere Affinität an Estrogenrezeptorpräparationen von Rattenprostata als an Estrogenrezeptorpräparationen von Rattenuterus und in vivo eine präferentielle Wirkung am Knochen im Vergleich zum Uterus aufweisen, deren Herstellung, ihre therapeutische Anwendung und pharmazeutischen Darreichungsformen, die die neuen Verbindungen enthalten. Bei den Verbindungen handelt es sich um 16a- und 16β-Hydroxy-estra,1,3,5(10)-estratriene, die am Steroid-Gerüst weitere Substituenten tragen sowie in den B-, C-und/oder D-Ringen eine oder mehrere zusätzliche Doppelbindungen aufweisen können.
Für die Behandlung von Fertilitätsstörungen der Frau, häufig verursacht durch chirurgisch, medikamentös oder anders bedingter ovarieller Dysfunktion eröffnen sich durch den Einsatz neuer selektiver Estrogene ebenfalls neue Therapiemöglichkeiten. Die in vitro Fertilitätsbehandlung ist ein seit mehr als 20 Jahren etabliertes Verfahren. Zahlreiche Methoden zur Behandlung der ovariell bedingten Infertilität mit exogenen Gonadotropinen sind bekannt. Durch Gabe von Gonadotropinen wie FSH (FSH = Follikel Stimulierendes Hormon) soll eine.

Stimulierung der Ovarien bewirkt werden, die eine gesunde Follikelreifung ermöglichen soll.

Der Follikel ist die funktionelle Einheit des Ovars und hat zwei Aufgaben: er beherbergt die Oozyte und schafft für diese so die Möglichkeit zum Wachstum und zur Reifung. Die Follikulogenese umfasst die Entwicklung eines ovariellen Follikels vom primordialen Stadium zu einem sich stetig vergrößernden Antralfollikel, der das letzte Stadium vor der Ovulation darstellt. Nur ein optimal entwickelter Antralfollikel kann eine ausgereifte Eizelle durch Ovulation freisetzen.
Patientinnen mit ovariell bedingter Infertilität (PCOS = Syndrom der Polycystischen Ovarien) leiden an einer gestörten Follikelreifung, welche sowohl mit hormonellen und ovulatorischen Störungen als auch mit unzureichend gereiften Eizellen verbunden ist. Die Anzahl der primären und sekundären Follikel ist hier ungefähr zweimal so hoch wie im normalen Ovar (Hughesden et al., Obstet. Gynecol. Survey **37**, *1982*, S. 59-77).
Es gibt Hinweise, dass die frühen Entwicklungsstadien der Follikulogenese (das betrifft die Entwicklung vom Primordial- zum Antralfollikel) Gondadotropinunabhängig sind. Es ist nicht eindeutig geklärt, wie groß der Einfluss bekannter parakriner und autokriner Faktoren auf die frühe Follikulogenese ist (Elvin et al., Mol. Cell Endocrinol. **13**, *1999,* S. 1035-1048; McNatty et al., J. Reprod. Fertil. Suppl., **54**, *1999,* S. 3-16).
Gonadotropine wie FSH sind hauptsächlich in den letzten Entwicklungsstadien der Follikulogenese an der Follikelreifung beteiligt, d.h. bei der Entwicklung vom frühen Antralfollikel zu einem reifen ovulationsfähigem Follikel.

Die in vivo und in vitro Infertilität wird vorzugsweise mit Gonadotropinen (FSH und Antiestrogenen) behandelt (White et al., J. Clin. Endocrinol. Metab. **81**, *1996*, S. 3821-3824). Bei der in vitro Fertilitätsbehandlung werden Oozyten aus präovulatorischen Antralfollikeln entnommen, um sie in vitro zu einer befruchtungsfähigen Eizelle heranreifen zu lassen. Nach der Befruchtung und der präembryonalen Entwicklung werden ein bis drei Embryos in den Uterus der Frau implantiert.

Die Behandlung mit exogenen Gonadotropinen ist in vielerlei Hinsicht von zahlreichen Risiken und Nebenwirkungen begleitet. Das größte Risiko besteht in einer Überstimulierung der Ovarien, welche in schweren Fällen eine ernsthafte Lebensgefahr darstellen kann (OHSS = Ovarian Hyperstimulation Syndrome). Weitere Nebeneffekte sind die hohen Kosten der in vitro Fertilitätsbehandlung, die von den Paaren getragen werden müssen. Negative Nebenwirkungen wie Gewichtszunahme, Aufgedunsenheit, Übelkeit, Erbrechen und ein bisher unbekanntes Langzeitrisiko, an Krebs zu erkranken, werden der Gonadotropin-Behandlung zugeschrieben.

Eine Methode, um die genannten Nachteile und Risiken zu vermeiden, wird darin gesehen, die Reifung und Stimulierung des follikulären Wachstums bei ovariell bedingter Infertilität in vivo mit einem geeigneten Wirkstoffen zu veranlassen, bevor eine Behandlung mit exogenen Gonadotropinen beginnt

### Estrogenrezeptor beta (ERβ)

Vor einigen Jahren wurde der Estrogenrezeptor-β (ERβ) als zweiter Subtyp des Estrogenrezeptors entdeckt (Kuiper et al. (1996), Proc. Natl. Acad. Sci. 93:5925-5930; Mosselman, Dijkema (1996) Febs Letters 392: 49-53; Tremblay et al. (1997), Molecular Endocrinology 11: 353-365). Das Expressionsmuster von ERβ unterscheidet sich von dem des ERα (Kuiper et al. (1996), Endocrinology 138: 863-870). So überwiegt ERβ gegenüber ERα in der Rattenprostata, während in Rattenuterus ERα gegenüber ERβ überwiegt. In den Ovarien wurden die höchsten Konzentrationen an ERβ mRNA gefunden (Couse et al. Endocrinology 138, 1997, S. 4612-4613).

Weitere Organsysteme mit vergleichsweise hoher ERβ-Expression umfassen den Knochen (Onoe Y et al., 1997, Endocrinology 138:4509-4512), das Gefäßsystem (Register TC, Adams MR 1998, J Steroid Molec Biol 64: 187-191), den Urogenitaltrakt (Kuiper GJM et al. 1997, Endocrinology 138: 863-870), den Gastrointestinaltrakt (Campbell-Thopson 1997, BBRC 240: 478-483), sowie die Testis (Mosselmann S et al. 1996 FEBS Lett. 392 49-53) einschließlich der Spermatiden (Shugrue et al. 1998, Steroids 63: 498-504). Die Gewebeverteilung legt nahe, dass Estrogene über ERβ Organfunktionen regulieren. Dass ERβ in dieser Hinsicht funktionell ist, ergibt sich auch durch Untersuchungen an ERα-(ERKO) bzw. ERβ-(βERKO)-Knockout-Mäusen: Ovariektomie bewirkt Knochenmasseverlust in ERKO-Mäusen, der durch Estrogensubstitution aufgehoben werden kann (Kimbro et al. 1998, Abstract OR7-4, Endocrine Society Meeting New- Orleans). Ebenso hemmt Estradiol in Blutgefäßen weiblicher ERKO-Mäuse die Gefäßmedia- und Glattmuskelzellproliferation (lafrati MD et al. 1997, Nature Medicine 3: 545-548). Diese protektiven Wirkungen von Estradiol erfolgen in der ERKO-Maus vermutlich über ERβ. Dass ERα und ERβ funktionell unterschiedlich wirken, wurde nach der erfolgreichen Erzeugung von αERKO- und βERKO-Mäusen bestätigt. Demzufolge spielt ERα eine wichtige Rolle im adulten Uterus, im Brustdrüsengewebe, bei der negativen Regulation der Gonadotropin-Aktivität, während ERβ hauptsächlich in die Vorgänge der ovariellen Physiologie eingebunden ist, insbesondere die der Follikulogenese und der Ovulation (Couse et al., Endocrine Reviews **20**, *1999*, S. 358-417).
Beobachtungen an βERKO-Mäusen liefern einen Hinweis auf eine Funktion von ERβ in Prostata und Blase: bei älteren männlichen Mäusen treten Symptome von Prostata- und Blasenhyperplasie auf (Krege JH et al. 1998, Proc Natl Acad Sci 95: 15677-15682). Außerdem weisen weibliche (Lubahn DB et al. 1993, Proc Natl Acad Sci 90:11162-11166) und männliche ERKO-Mäuse (Hess RA et al. 1997, Nature 390: 509-512) sowie weibliche βERKO-Mäuse (Krege JH, 1998, Proc Natl Acad Sci 95: 15677-15682) Fertilitätsstörungen auf. Hierdurch wird die wichtige Funktion von Estrogenen hinsichtlich Aufrechterhaltung von Testis- und Ovarfunktion sowie Fertilität belegt.

Eine selektive Estrogenwirkung auf bestimmte Zielorgane könnte aufgrund der unterschiedlichen Gewebe- bzw. Organverteilung der beiden Subtypen des ERs durch subtypspezifische Liganden erreicht werden. Substanzen mit Präferenz für ERβ verglichen mit ERα im in vitro Rezeptorbindungstest wurden von Kuiper et al. beschrieben (Kuiper et al. (1996), Endocrinology 138: 863-870). Eine selektive Wirkung von subtypspezifischen Liganden des Estrogenrezeptors auf estrogensensitive Parameter in vivo wurde bisher nicht gezeigt.

Aufgabe der vorliegenden Erfindung ist es, Verbindungen bereitzustellen, die in vitro eine Dissoziation hinsichtlich der Bindung an Estrogenrezeptorpräparationen von Rattenprostata und Rattenuterus aufweisen. Die Verbindungen sollen in vitro eine höhere Affinität an Estrogenrezeptorpräparationen von Rattenprostata als an Estrogenrezeptorpräparationen von Rattenuterus zeigen.
Die ERβ-spezifischen Verbindungen sollen in vivo eine Profertilitätswirkung im Ovar hervorrufen. Gleichzeitig sollen die Verbindungen eine Dissoziation hinsichtlich Ovar- im Vergleich zur Uteruswirkung aufweisen. Die erfindungsgemäßen Verbindungen sollen eine gewisse protektive Wirksamkeit gegen hormondefizienz-bedingten Knochenmasseverlust im Vergleich zur uterusstimulierenden Wirkung besitzen.

Im weiteren Sinne soll durch die vorliegende Erfindung eine Struktur-Wirkungsbeziehung zur Verfügung gestellt werden, die den Zugang zu Verbindungen gestattet, die das oben formulierte pharmakologische Profil besitzen. Die erfindungsgemäßen Verbindungen sollen bei ovariell bedingter Infertilität im Ovar eine Fertilitätsverbesserung bei gleichzeitig geringerer Wirksamkeit am Uterus bewirken.

Erfindungsgemäß gelöst wird die vorstehende Aufgabe durch die Bereitstellung der 9α-substituierten Estra-1,3,5(10)-trienderivate der allgemeinen Formel I 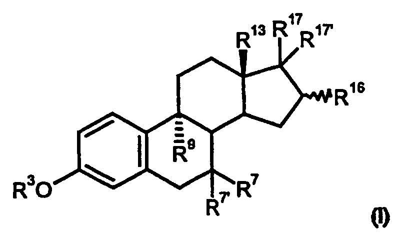 worin die Reste R³,R⁷,R^{7'},R⁸,R¹³,R¹⁶ sowie R¹⁷ und R^{17'} unabhängig voneinander folgende Bedeutung besitzen:
- R³: ein Wasserstoffatom oder eine Gruppe R¹⁸, worin
R¹⁸ ein gerad-oder verzweigtkettiger, gesättigter oder ungesättigter Kohlenwasserstoffrest mit bis zu 6 Kohlenstoffatomen, eine Trifluormethylgruppe, ein Aryl-, Heteroaryl- oder Aralkylrest, ein substituierter Aryl-, Heteroarylrest mit ein Methyl-, Ethyl-, Trifluormethyl-, Pentafluorethyl-, Trifluormethylthio-, Methoxy-, Ethoxy-, Nitro-, Cyano-, Halogen- (Fluor, Chlor, Brom, Iod), Hydroxy-, Amino-, Mono(C₁₋₈-alkyl)- oder Di(C₁₋₈-alkyl) amino, wobei beide Alkylgruppen identisch oder verschieden sind, Di (aralkyl) amino, wobei beide Aralkylgruppen identisch oder verschleden sind, Carboxyl, Carboxyalkoxy, C₁-C₂₀-Acyl oder C₁-C₂₀-Acyloxy-Gruppen als Substituenten, ein Acylrest COR¹⁹, worin R¹⁹ ein, gerad-oder verzweigtkettiger, gesättigter oder bis zu dreifach ungesättigter, teilweise oder vollständig halogenierter Kohlenwasser- stoffrest mit bis zu 10 Kohlenstoffatomen ist, oder
R¹⁸ eine Gruppe R²⁰SO₂-bedeutet, worin
R²⁰ eine R²¹R²²N - Gruppe ist, wobei R²¹ und R²² unabhängig voneinander ein Wasserstoffatom, einen C₁-C₆ - Alkylrest, eine Gruppe C(O)R²³, worin R²³ einen gerad- oder verzweigtkettigen, gesättigten oder bis zu dreifach ungesättigten, teilweise oder vollständig halogenierten Kohlenwasserstoffrest mit bis zu 10 Kohlenstoffatomen, einen Cyclopropyl-, -butyl-,-pentyl-, -hexyl- oder -heptylgruppe, einen C₄-C₁₅-Cycloalkyl-alkylrest mit einem 3 bis 7 Kohlenstoffatome im Cycloalkylteil und mit einem Alkylteil bis zu 8 kohlenstoffatome oder einen Aryl-, Heteroaryl- oder Aralkylrest darstellt, oder ein substituierter Aryl-, Heteroarylrest mit ein Methyl, Ethyl-, Trifluormethyl-, Pentafluorethyl-, Trifluormethylthio-, Methoxy-, Ethoxy-, Nitro-, Cyano-, Halogen- (Fluor, Chlor, Brom, Iod), Hydroxy-, Amino-, Mono(C₁₋₈-alkyl)- oder Di(C₁₋₈-alkyl) amino, wobei beide Alkylgruppen identisch oder verschieden sind, Di (aralkyl) amino, wobei beide Aralkylgruppen identisch oder verschieden sind, Carboxyl, Carboxyalkoxy, C₁-C₂₀-Acyl oder C₁-C₂₀-Acyloxy-Gruppen als Substituenten oder, zusammen mit dem N-Atom, einen Polymethyleniminorest mit 4 bis 6 C-Atomen oder einen Morphollnorest, bedeuten,
R⁷ und R^{7'} jeweils unabhängig voneinander ein Wasserstoffatom oder ein Halogenatom,
R⁹ ein gerad-oder verzweigtkeltiger Alkenyl-oder Alkinylrest mit 2 bis 6 Kohlenstoffatomen, der teilweise oder vollständig fluoriert sein kann, oder ein Ethinyl-oder Prop-1-inylrest
R¹³ eine Methylgruppe oder eine Ethylgruppe,
R¹⁸ eine Hydroxygruppe oder eine Gruppe R¹⁸O-, R²⁰SO₂- oder OC(O)R²³ mit R¹⁸, R²⁰ und R²³ jeweils in der unter R³ angegebenen Bedeutung,
R¹⁷ und R^{17'} jeweils unabhängig voneinander ein Wasserstoffatom oder ein Halogenatom.

R¹⁸ kann jeweils in der α-oder β-Position stehen.

Gemäß einer Variante der Erfindung werden Gonatrienderivate bevorzugt, worin R⁷ und R^{7'} ein Wasserstoffatom, R⁹ eine Vinyl-, Ethinyl- oder Prop-1-inylgruppe, R¹⁹ eine Hydroxygruppe und R¹⁷ und R^{17'} jeweils ein Wassenstonatom sind.

Weiterhin sind folgende Kombinationen aus Halogensubstitution, vorzugsweise Fluor, am C-Atom 7 und 17 bevorzugt 7-mono oder 7-di und R¹⁷ sowie R^{17'} jeweils ein Wasserstoff, 17-mono oder 17-di und R⁷ sowie R^{7'} jeweils ein Wsssentoff sowie 7-mono/ 17-mono, 7-mono/ 17-di, 7-di/ 17-mono, 7-di/ 17-di. Die 7 α- bzw. die 17β-Position ist bei den Monofluorverbindungen bevorzugt.

Eine weitere Variante der Erfindung sieht insbesondere Verbindungen vor, worin R¹⁸ für eine Gruppe R¹⁸O- oder R²⁰SO₂-O- mit R¹⁸ und R²⁰ jeweils in der unter R³ angegebenen Bedeutung steht.

Bevorzugt gemäß vorliegender Erfindung sind die folgenden Verbindungen
9α-Vinyl-estra-1,3,5(10)-trien-3,16α-diol
9α-Allyl-estra-1,3,5(10)-trien-3,16α-diol
18a-Homo-9α-vinyl-estra-1,3,5(10)-trien-3,16α-diol
18a-Homo-9α-allyl-estra-1,3,5(10)-trien-3,16α-diol
3-Methoxy-9α-vinyl-estra-1,3,5(10)-trien-16α-ol
9α-Allyl-3-methoxy-estra-1,3,5(10)-trien-16α-ol
18a-Homo-3-methoxy-9α-vinyl-estra-1,3,5(10)-trien-16α-ol
18a-Homo-9α-allyl-3-methoxy-estra-1,3,5(10)-trien-16α-ol
9α-(2',2'-Difluorvinyl)-estra-1,3,5(10)-trien-3,16α-diol
9α-(2',2'-Difluorvinyl)-3-methoxy-estra-1,3,5(10)-trien-16α-ol
16α-Hydroxy-9α-vinyl-estra-1,3,5(10)-trien-3yl-sulfamat
9α-Allyl-16α-hydroxy-estra-1,3,5(10)-trien-3yl-sulfamat
18a-Homo-16α-hydroxy-9α-vinyl-estra-1,3,5(10)-trien-3yl-sulfamat
18a-Homo-9α-allyl-16α-hydroxy-estra-1,3,5(10)-trien-3yl-sulfamat
9α-Vinyl-estra-1,3,5(10)-trien-3,16α-diyl-disulfamat
9α-Allyl-estra-1,3,5(10)-trien-3,16α-diyl-disulfamat
18a-Homo-9α-vinyl-estra-1,3,5(10)-trien-3,16α-diyl-disulfamat
18a-Homo-9α-allyl-estra-1,3,5(10)-trien-3,16α-diyl-disulfamat
16α-Hydroxy-9α-vinyl-estra-1,3,5(10)-trien-3yl-(N-acetyl)-sulfamat
9α-Allyl-16α-hydroxy-estra-1,3,5(10)-trien-3yl-(N-acetyl)-sulfamat
18a-Homo-16α-hydroxy-9α-vinyl-estra-1,3,5(10)-trien-3yl-(N-acetyl)-sulfamat
18a-Homo-9α-allyl-16α-hydroxy-estra-1,3,5(10)-trien-3yl-(N-acetyl)-sulfamat
9α-(Prop-(Z)-enyl)-estra-1,3,5(10)-trien-3,16α-diol
9α-(n-Propyl)-estra-1,3,5(10)-trien-3,16α-diol
9α-Ethinyl-estra-1,3,5(10)-trien-3,16α-diol
9α-Vinyl-estra-1,3,5(10)-trien-3,16α-diol-diacetat
18a-Homo-9α-vinyl-estra-1,3,5(10)-trien-3,16α-diol-diacetat
16α-Valeroyloxy-9α-vinyl-estra-1,3,5(10)-trien-3-ol
16α-Acetoxy-9α-vinyl-estra-1,3,5(10)-trien-3-ol
18a-Homo-16α-acetoxy-9α-vinyl-estra-1,3,5(10)-trien-3-ol
7α-Fluor-9α-vinyl-estra-1,3,5(10)-trien-3,16α-diol
7α-Fluor-9α-allyl-estra-1,3,5(10)-trien-3,16α-diol
17β-Fluor-9α-vinyl-estra-1,3,5(10)-trien-3,16α-diol
17β-Fluor-9α-allyl-estra-1,3,5(10)-trien-3,16α-diol
18a-Homo-7α-fluor-9α-vinyl-estra-1,3,5(10)-trien-3,16α-diol
18a-Homo-7α-fluor-9α-allyl-estra-1,3,5(10)-trien-3,16α-diol
18a-Homo-17β-fluor-9α-vinyl-estra-1,3,5(10)-trien-3,16α-diol
18a-Homo-17β-fluor-9α-allyl-estra-1,3,5(10)-trien-3,16α-diol
Weitere Ausgestaltungsmöglichkeiten der vorliegenden Erfindung ergeben sich aus den Unteransprüchen.

Der Kohlenwasserstoffrest R¹⁸ ist beispielsweise ein Methyl, Ethyl, Propyl, Isopropyl, Butyl, Isobutyl, tert.-Butyl, Pentyl, Isopentyl, Neopentyl, Hexylrest.

Die Alkoxygruppen OR¹⁸ in den Verbindungen der allgemeinen Formel I können jeweils 1 bis 6 Kohlenstoffatome enthalten, wobei Methoxy-, Ethoxy- Propoxy-Isopropoxy- und t-Butyloxygruppen bevorzugt sind.

Vertreter für die C₁-C₅-Alkylreste R²¹ und R²² sind Methyl, Ethyl, Propyl, Isopropyl, Butyl, Isobutyl, tert.-Butyl, Pentyl, Isopentyl, Neopentyl.

Als Vertreter für gerad- oder verzweigtkettige Kohlenwasserstoffreste R²³ mit 1 bis max. 10 Kohlenstoffatomen sind beispielsweise Methyl, Ethyl, Propyl, Isopropyl, Butyl, Isobutyl, tert.-Butyl, Pentyl, Isopentyl, Neopentyl, Heptyl, Hexyl und Decyl zu nennen; Methyl, Ethyl, Propyl und Isopropyl sind bevorzugt.

Als C₃-C₇-Cycloalkylgruppe ist eine Cyclopropyl-, -butyl-, -pentyl-, -hexyl- oder -heptylgruppe zu nennen.

Ein C₄-C₁₅-Cycloalkylalkylrest weist 3 bis 7 Kohlenstoffatome im Cycloalkylteil auf; typische Vertreter sind die direkt vorstehend genannten Cycloalkylgruppen. Der Alkylteil weist bis zu 8 Kohlenstoffatome auf.
Als Beispiele für einen C₄-C₁₅-Cycloalkylalkylrest seien die Cyclopropylmethyl-, Cyclopropylethyl-, Cyclopentylmethyl-, Cyclopentylpropylgruppe etc. genannt.

Bei einem Arylrest handelt es sich im Sinne der vorliegenden Erfindung um einen Phenyl-, 1- oder 2-Naphthylrest; der Phenylrest ist bevorzugt.

Aryl schließt immer auch einen Heteroarylrest mit ein. Beispiele für einen Heteroarylrest sind der 2-, 3- oder 4-Pyridinyl-, der 2- oder 3-Furyl-, der 2- oder 3-Thienyl-, der 2- oder 3-Pyrrolyl, der 2-, 4- oder 5-Imidazolyl-, der Pyrazinyl-, der 2-, 4- oder 5-Pyrimidinyl- oder 3- oder 4-Pyridazinylrest.

Als Substituenten, die an einem Aryl- oder Heteroarylrest vorhanden sein können, seien zum Beispiel ein Methyl-, Ethyl-, Trifluormethyl- Pentafluorethyl-, Trifluormethylthio-, Methoxy-, Ethoxy-, Nitro-, Cyano-, Halogen- (Fluor, Chlor, Brom, lod), Hydroxy-, Amino-, Mono(C₁₋₈-alkyl)- oder Di(C₁₋₈-alkyl)amino, wobei beide Alkylgruppen identisch oder verschieden sind, Di(aralkyl)amino, wobei beide Aralkylgruppen identisch oder verschieden sind, Carboxyl, Carboxyalkoxy, C₁-C₂₀-Acyl oder C₁-C₂₀-Acyloxy-Gruppen erwähnt.

Bei einem Aralkylrest handelt es sich um einen Rest, der im Ring bis 14, bevorzugt 6 bis 10, C-Atome und in der Alkylkette 1 bis 8, bevorzugt 1 bis 4, C-Atome enthält. So kommen als Aralkylreste beispielsweise in Betracht Benzyl, Phenylethyl, Naphthylmethyl, Naphthylethyl, Furylmethyl, Thienylethyl, Pyridylpropyl.

Die Alkylgruppen bzw. Kohlenwasserstoffreste können teilweise oder vollständig substituiert sein durch 1-5 Halogenatome, Hydroxygruppen oder C₁-C₄-Alkoxygruppen.

Mit einem C₂-C₆-Alkenylrest ist in erster Linie ein Vinyl- oder Allylrest gemeint.

Unter einem C₂-C₆-Alkinylrest ist bevorzugt ein Ethinyl- oder Prop-1-inylrest zu verstehen.

C₁₋₁₀-Acylreste bedeuten beispielsweise Acetyl, Propionyl, Butyryl, Valeroyl, Isovaleroyl, Pivaloyl, Hexanoyl, Octyl, Nonyl, Decanoyl.

Eine oder beide Hydroxylgruppen an den C-Atomen 3 und 16 können mit einer aliphatischen, gerad- oder verzweigtkettigen, gesättigten oder ungesättigten C₁-C₁₄-Mono- oder Polycarbonsäure oder einer aromatischen Carbonsäure verestert sein.
Als derartige Carbonsäuren zur Veresterung kommen beispielsweise in Betracht:
Monocarbonsäuren: Ameisensäure, Essigsäure, Propionsäure, Buttersäure, Isobuttersäure, Valeriansäure, Isovaleriansäure, Pivalinsäure, Laurinsäure, Myristinsäure, Acrylsäure, Propionsäure, Methacrylsäure, Crotonsäure, Isocrotonsäure, Ölsäure, Elaidinsäure.
Die Veresterung mit Essigsäure, Valeriansäure oder Pivalinsäure ist bevorzugt. Dicarbonsäuren: Oxalsäure, Malonsäure, Bemsteinsäure, Glutarsäure, Adipinsäure, Pimelinsäure, Korksäure, Azelainsäure, Sebacinsäure, Maleinsäure, Fumarsäure, Muconsäure, Citraconsäure, Mesaconsäure.
Aromatische Carbonsäuren: Benzoesäure, Phthalsäure, Isophthalsäure, Terephthalsäure, Naphthoesäure, o-, m- und p-Toluylsäure, Hydratropasäure, Atropasäure, Zimtsäure, Nicotinsäure, Isonicotinsäure. Die Veresterung mit Benzoesäure ist bevorzugt.

Die erfindungsgemäßen Ester der 9α-substituierten Estratriene weisen als Prodrugs Vorteile gegenüber den unveresterten Wirkstoffen hinsichtlich ihres Applikationsmodus, ihrer Wirkungsart, Wirkungsstärke und Wirkungsdauer auf.

Pharmakokinetische und pharmakodynamische Vorteile weisen insbesondere die erfindungsgemäßen Sulfamate der 9α-substituierten Estratriene auf. Diesbezügliche Effekte wurden bereits bei anderen Steroid-Sulfamaten beschrieben (J. Steroid Biochem. Molec. Biol, 55, 395 - 403 (1995); Exp. Opinion Invest. Drugs 7, 575 - 589 (1998)).

In der vorliegenden Patentanmeldung werden Steroide, denen das 9α-substituierte Estra-1,3,5(10)-trien-Gerüst zugrunde liegt, zur Behandlung von Estrogenrezeptor β-vermittelten Störungen und Zuständen als selektive Estrogene beschrieben, die in vitro Dissoziation hinsichtlich ihrer Bindung an Estrogenrezeptorpräparationen von Rattenprostata und Rattenuterus und die in vivo vorzugsweise eine Dissoziation hinsichtlich Ovar- im Vergleich zu Uteruswirkung aufweisen. Die Verbindungen besitzen außerdem eine gewisse protektive Wirkung gegen hormondefizienz bedingten Knochenmasseverlust.

Es wurde gefunden, dass 9α-substituierten Estra-1,3,5(10)-triene gemäß allgemeiner Formel I als selektive Estrogene zur Behandlung verschiedener Zustände und Störungen geeignet sind, die durch einen höheren Gehalt an Estrogenrezeptor β als Estrogenrezeptor α im entsprechenden Zielgewebe oder -organ gekennzeichnet sind.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung der Verbindungen der allgemeinen Formel I zur Herstellung von Arzneimitteln, insbesondere für die nachstehend genannten Indikationen.

Die hier beschriebenen neuen selektiven Estrogene können als Einzelkomponente in pharmazeutischen Zubereitungen oder in Kombination insbesondere mit Gestagenen eingesetzt werden. Besonders bevorzugt ist die Kombination der selektiven Estrogene mit ERα-selektiven Antiestrogenen, die peripherselektiv wirksam sind, d.h. die die Bluthirnschranke nicht passieren, sowie mit selektiven Estrogenrezeptormodulatoren (SERM). Die erfindungsgemäßen ERβ-selektiven Verbindungen können insbesondere für die Herstellung von Arzneimitteln zur Behandlung von Fertilitätsstörungen, zur Prävention und Therapie der Prostatahyperplasie, zur Prävention und Behandlung von hormondefizienz bedingten Stimmungsschwankungen bei Frau und Mann sowie zur Anwendung in der Hormonersatztherapie (HRT) bei Mann und Frau verwendet werden.

Ein therapeutisches Produkt, enthaltend ein Estrogen und ein reines Antiestrogen für gleichzeitige, sequentielle oder getrennte Anwendung für die selektive Estrogentherapie perimenopausaler oder postmenopausaler Zustände ist bereits in der EP-A 0 346 014 beschrieben.

Die Substanzen und die sie enthaltenden Pharmaka sind aufgrund ihrer Wirkungsdissoziation am Ovar im Vergleich zur Uteruswirkung besonders geeignet für die Behandlung bei chirurgisch, medikamentös oder anders bedingter ovarieller Dysfunktion wie weibliche Infertilität zur Stimulierung der Follikulogenese zur alleinigen Behandlung im Sinne einer Fertilitätsverbesserung, zur Unterstützung der in vitro Fertilitätsbehandlung (IVF) in Verbindung mit einer in vivo Behandlung und zur Behandlung von ovariell bedingten Störungen im Alter ("späte Fertilität") sowie zur Behandlung von hormondefizienz bedingten Beschwerden.
Die Substanzen sind auch zur Therapie ovarieller Erkrankungen wie Polycystisches Ovar Syndrom, POF (Premature Ovarian Failure)-Syndrom und Ovulationsstörungen geeignet.

Schließlich können die Verbindungen der allgemeinen Formel I in Verbindung mit Selektiven Estrogenrezeptormodulatoren (SERM) bsplw. Raloxifen verwendet werden, und zwar insbesondere zur Verwendung in der Hormonersatztherapie (HRT) und zur Behandlung gynäkologischer Störungen.

Auch als Einzelkomponente sind die Substanzen geeignet für die Behandlung peri- und postmenopausaler Beschwerden insbesondere Hitzewallungen, Schlafstörungen, Reizbarkeit, Stimmungsschwankungen, Inkontinenz, Vaginalatrophie und hormondefizienzbedingter Gemütserkrankungen. Ebenso sind die Substanzen für die Hormonsubstitution und die Therapie von hormondefizienz bedingten Beschwerden bei chirurgisch, medikamentös oder anders bedingter ovarieller Dysfunktion geeignet.

Die Substanzen sind außerdem zur Prophylaxe gegen hormondefizienzbedingten Knochenmasseverlust und Osteoporose, zur Vorbeugung gegen Herzkreislauferkrankungen, insbesondere Gefäßerkrankungen wie Atherosklerose, Bluthochdruck und zur Vorbeugung gegen hormondefizienzbedingte neurodegenerative Erkrankungen, wie Alzheimersche Krankheit, sowie hormondefizienzbedingte Beeinträchtigung von Gedächtnis- und Lemfähigkeit, einsetzbar.
Weiterhin sind die Substanzen als Wirkstoff in Präparaten zur Behandlung von entzündlichen und Erkrankungen des Immunsystems, insbesondere Autoimmunerkrankungen, wie z.B. Rheumatoide Arthritis, Multiples Sklerose, Lupus, Morbus Crohn und anderer entzündlicher Darmerkrankungen, entzündlicher Erkrankungen der Haut, wie Psoriasis, sowie zur Behandlung der Endometriose einsetzbar.
Weiterhin sind die Substanzen wirksam gegen entzündliche Erkrankungen der Atemwege, der Lunge und Bronchen, wie z.B. Asthma.
Das Medikament eignet sich zur Therapie und Prophylaxe estrogendefizienzbedingter Erkrankungen sowohl von Frauen als auch Männern.

Die Verbindungen sind bei Männern insbesondere geeignet zur Therapie von hormondefizienzbedingtem Knochenmasseverlust und Osteoporose, zur Vorbeugung gegen Herzkreislauferkrankungen, insbesondere Gefäßerkrankungen wie Atherosklerose, Bluthochdruck und zur Vorbeugung gegen hormondefizienzbedingte neurodegenerative Erkrankungen, wie Alzheimersche Krankheit, sowie hormondefizienzbedingte Beeinträchtigung von Gedächtnis- und Lernfähigkeit, einsetzbar, sowie zur Prevention und Therapie der Prostatahyperplasie geeignet.

Die Substanzen können zur Prophylaxe und Therapie altersbedingter Dysfunktionen oder Erkrankungen des Mannes eingesetzt werden. Insbesondere sind sie einsetzbar zur Prävention und Behandlung eines alterbedingten Abfalls von Androgenen, wie Testosteron und DHEA, sowie von Wachstumshormon.

Weiterhin ist das Medikament zur Behandlung von entzündlichen und Erkrankungen des Immunsystems, insbesondere Autoimmunerkrankungen beim Mann, wie z.B. Rheumatoide Arthritis, MS (Multiples Sklerose) und Morbus Crohn und andere entzündliche Darmerkrankungen, sowie entzündliche Erkrankungen der Atemwege, der Lunge und der Bronchen einsetzbar. Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 µg/kg - 100 mg/kg Körpergewicht, vorzugsweise 0,04 µg/kg - 1 mg/kg Körpergewicht, je Tag betragen.
Beim Menschen entspricht dies einer Dosis von 0,8 µg bis 8 g, vorzugsweise 3,2 µg bis 80 mg, täglich.

Eine Dosiseinheit enthält erfindungsgemäß 1,6 µg bis 2000 mg einer oder mehrerer Verbindungen der allgemeinen Formel I.

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2, 1961, Seite 72 u. ff.: Dr. H. P. Fiedler, Lexikon der Hilfsstoffe für Pharmazie, Kosmetik und angrenzende Gebiete, Cantor KG. Aulendorf in Württemberg 1971.

Die Verbindungen können oral oder parenteral, beispielsweise intraperitoneal, intramuskulär, subkutan oder perkutan verabreicht werden. Die Verbindungen können auch in das Gewebe implantiert werden.
Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.
Zur parenteralen Verabreichung können die Wirkstoffe in einem physiologisch verträglichen Verdonnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnussöl, Baumwollsamenöl, Sojabohnenöl, Rizinusöl und Sesamöl.
Die Verbindungen lassen sich auch in Form einer Depotinjektion oder eines Implantatpräparats anwenden, die so formuliert sein können, dass eine verzögerte Wirkstoff-Freigabe ermöglicht wird.
Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Für die Herstellung von mit aktiven Verbindungen der allgemeinen Formel I beladenen Intravaginal- (z.B. Vaginalringe) oder Intrauterinsystemen (z.B.
Pessare, Spiralen, IUSs, Mirena®) für die lokale Verabreichung eignen sich verschiedene Polymere wie zum Beispiel Silikonpolymere, Ethylenvinylacetat, Polyethylen oder Polypropylen.

Um eine bessere Bioverfügbarkeit des Wirkstoffes zu erreichen, können die Verbindungen auch als Cyclodextrinclathrate formuliert werden. Hierzu werden die Verbindungen mit α-, β- oder γ-Cyclodextrin oder Derivaten von diesen umgesetzt (PCT/EP95/02656).

Erfindungsgemäß können die Verbindungen der allgemeinen Formel I auch mit Liposomen verkapselt werden.

### Methoden

### Estrogenrezeptorbindungsstudien:

Die Bindungsaffinität der neuen selektiven Estrogene wurde in Kompetitionsexperimenten unter Verwendung von ³H-Estradiol als Ligand an Estrogenrezeptorpräparationen von Rattenprostata und Rattenuterus getestet Die Präparation des Prostatacytosols und der Estrogenrezeptortest mit dem Prostatacytosol wurde, wie von Testas et al. (1981) beschrieben, durchgeführt (Testas J. et al., 1981, Endocrinology 109: 1287-1289).
Die Präparation von Rattenuteruscytosol, sowie der Rezeptortest mit dem ERhaltigen Cytosol wurden prinzipiell durchgeführt wie von Stack und Gorski (1985) beschrieben (Stack, Gorski 1985, Endocrinology 117, 2024-2032) mit einigen Modifikationen wie bei Fuhrmann et al. (1995) beschrieben (Fuhrmann U. et al. 1995, Contraception 51: 45-52).

Die hier beschriebenen Substanzen weisen höhere Bindungsaffinitäten zum Estrogenrezeptor aus Rattenprostata als zum Estrogenrezeptor aus Rattenuterus auf. Dabei wird davon ausgegangen, dass ERβ gegenüber ERα in der Rattenprostata, im Rattenuterus ERα gegenüber ERβ überwiegt. Tabelle 1 zeigt, dass das Verhältnis der Bindung an Prostata- und Uterusrezeptor qualitativ dem Quotienten der relativen Bindungsaffinität (RBA) an humanen ERβ und ERα von Ratte (nach Kuiper et al. (1996), Endocrinology 138: 863-870) entspricht (Tabelle 1).

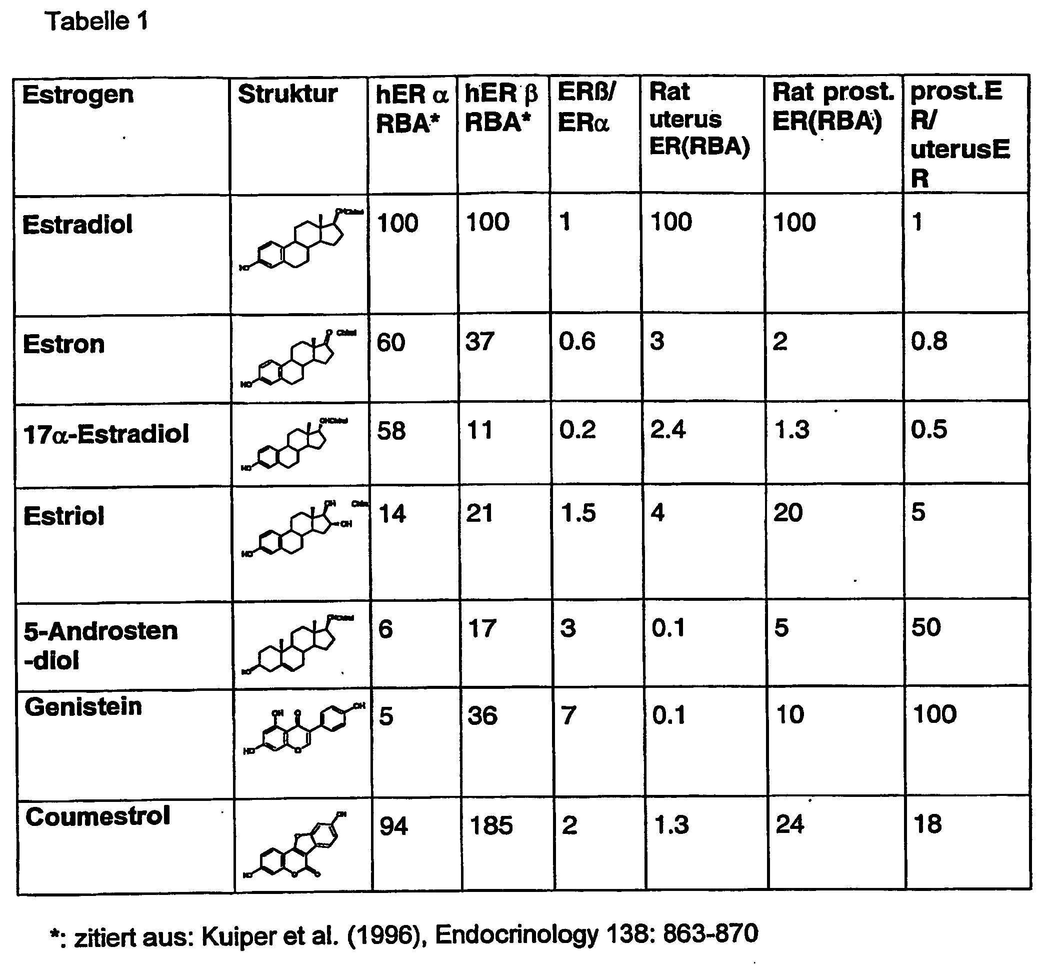

Tabelle 2 zeigt die Ergebnisse für 4 der erfindungsgemäßen 9α-Vinyl-estra-1,3,5(10)-trien-3,16α-diol-Derivate (Verbindungen **1;2;4;5**)

**Tabelle 2**

| **Verbindung** | **RBA** **Rattenuterus** | **RBA** **Rattenprostata** |
|---|---|---|
| 9α-Vinyl-estra-1,3,5(10)-3,16α-diol (1) | 1.2 | 100 |
| 9α-Vinyl-estra-1,3,5(10)-17F-3,16α-diol (2) | 2 | 200 |
| 9α-Di-F-Vinyl-estra-1,3,5(10)-3,16α-diol (4) | 0.2 | 4 |
| 9α-Di-F-Vinyl-estra-1,3,5(10)-13-Methyl-3,16α-diol (5) | 0.2 | 6 |

Die erfindungsgemäßen Verbindungen **1; 2; 4; 5** zeigen eine höhere Bindungsaffinität am Estrogenrezeptor aus Rattenprostata als am Estrogenrezeptor aus Rattenuterus.

Weiterhin wurde die Prädiktivität des Prostata-ER versus Uterus-ER-Testsystems hinsichtlich gewebeselektiver Wirkung durch in vivo Untersuchungen bestätigt. Substanzen mit Präferenz für Prostata-ER sind in vivo vorzugsweise hinsichtlich Ovar- und Uterus- sowie Hypophysenwirkung zugunsten der Wirkung am Ovar dissoziiert.

### Untersuchungen zur Wirkungsdissoziation Ovar/ Uterus und Hypophyse

Die Untersuchungen hinsichtlich der Wirkung auf das Uteruswachstum und die Ovulation (indirekter Effekt durch Beeinflussung der Sekretion von Hypophysenhormonen) werden an adulten weiblichen Ratten (Körpergewicht 220-250 g) durchgeführt. Die Substanzen werden viermal an vier aufeinanderfolgenden Tagen subkutan appliziert. Die erste Applikation erfolgt im Metestrus. Einen Tag nach der letzten Applikation erfolgt die Autopsie. Es wird die Anzahl der Eizellen in der Tube (Effekt auf die Ovulation) sowie das Uterusgewicht bestimmt.

Während Estradiol eine dosisabhängige Ovulationshemmung und eine Erhöhung des Uterusgewichts mit einer ED₅₀ von 0.004 mg/ kg Körpergewicht bewirkt, übt die erfindungsgemäße Substanz **1** bis zu einer Dosis von 0.4 mg/kg Körpergewicht keinen Effekt auf Ovulation und Uterusgewicht aus.

### Untersuchungen am Ovar:

Die Substanzen wurdenin vivo an hypophysektomierten juvenilen Ratten geprüft. In einer Modifikation dieser operativen Methode wird den Tieren ein GnRH-Antagonisten appliziert. Es wird geprüft, ob die Substanz die Follikelproliferation (Reifung) im Ovar stimuliert. Messparameter ist das Ovargewicht.
Jeweils fünf Tiere (Körpergewicht 40-50 g) werden den Behandlungsgruppen randomisiert zugeordnet. Die Tiere werden in Makrolonkäfigen in klimatisierten Räumen mit Lichtprogramm (10 h dunkel, 14 h hell) mit einer Standarddiät (Altromin) ad libitum ernährt und mit angesäuertem Leitungswasser getränkt. Für die s.c.-Applikation werden die Testsubstanz sowie die Kontrollsubstanz (Estradiol E2) in BenzylbenzoatlRhizinusöl (1+4 v/v) gelöst.

Die juvenilen weibliche Ratten werden entweder an Tag 0 hypophysektomiert und von Tag 1 bis Tag 4 mit Estradiol, der erfindungsgemäßen Verbindung **1** bzw. 2 oder mit einem Vehikel (Rizinusöl/Benzylbenzoat) subkutan behandelt (Applikation 1x täglich). In der modifizierten Version der Methode bekommen die Tiere gleichzeitig mit der Verbindung **2** bzw. dem Vehikel und der Kontrollsubstanz Estradiol Ober vier Behandlungstage 0,5 mg/Tier/Tag Cetrorelix verabreicht. In beiden Fällen werden die Tiere 24 h nach der letzten Applikation getötet und die Ovargewichte bestimmt.

0,5 mg/Tier/Tag der Verbindung **1** über 4 Tage subkutan appliziert bewirken bei hypophysektomierten Tieren eine vergleichbare Erhöhung des Ovargewichts wie Estradiol mit einer Dosis von 0,1 mg/Tier/Tag. Das Vehikel bewirkt keinen Effekt.

Damit zeigt die erfindungsgemäße Substanz **1** eine Wirkungsdissoziation am Ovar im Vergleich zur Uterus- und Hypophysenwirkung und ist aufgrund ihrer follikelstimulierenden Wirkung hervorragend für die bevorzugte Indikation, die Behandlung der weiblichen Infertilität, geeignet.
Bei den GnRH-Antagonist-behandelten Tieren zeigen bereits Konzentrationen von 0,1 und 0,3 mg/Tier/Tag der Verbindung **2** am Ovar die gleiche Wirkung wie die eingesetzte Dosis von 1mg/Tier/Tag Estradiol (Abb. 1). Selbst niedrigere Dosierung (0,01, 0,03 mg/Tier/Tag) zeigen eine Ovarwirkung und können den antagonistischen Effekt von Cetrorelix aufheben (Abb. 2).

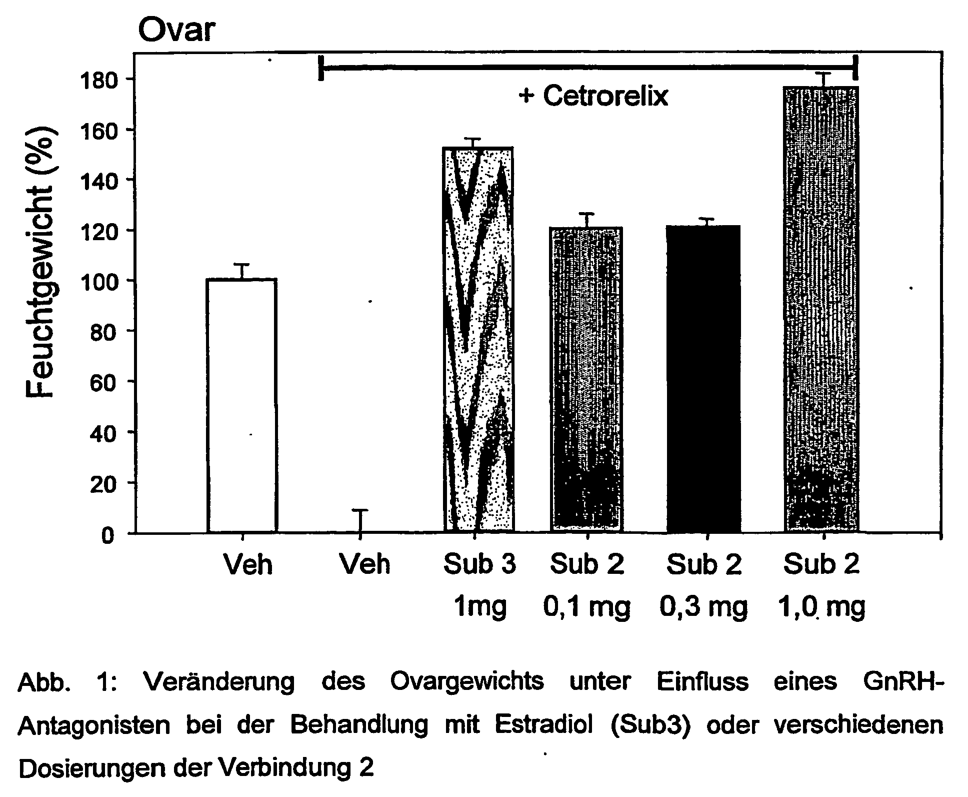

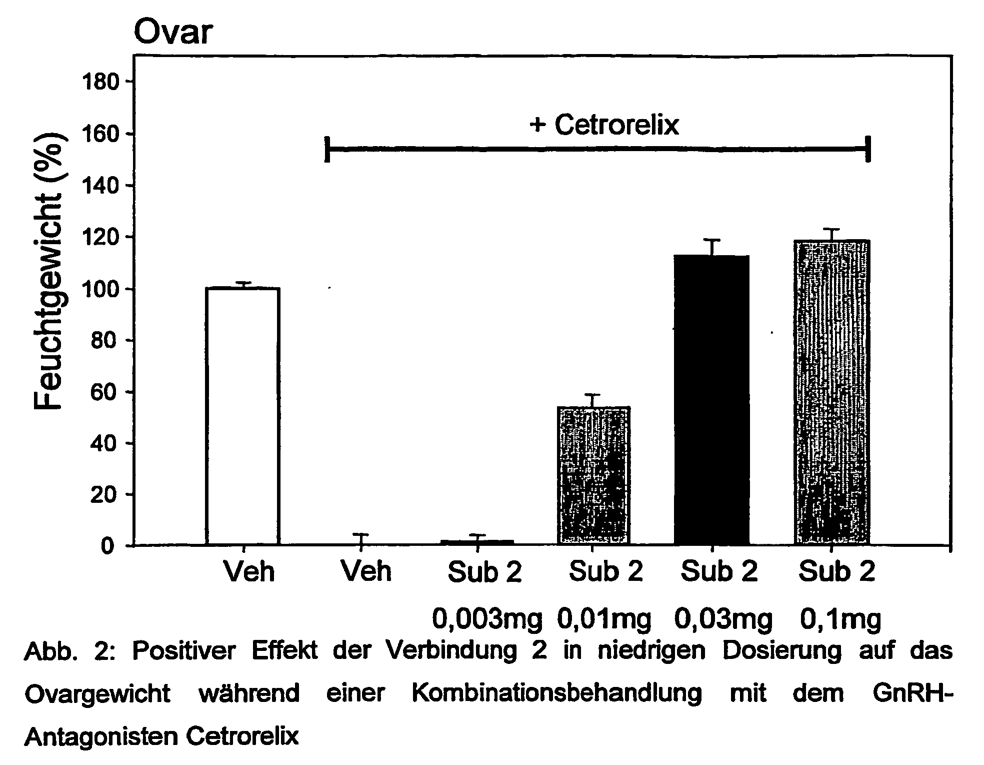

Damit zeigt auch die erfindungsgemäße Substanz **2** eine deutlich positive Wirkung am Ovar, indem sie die Follikelanreifung stimuliert und deshalb ebenfalls für die Behandlung weiblicher Sub- oder Infertilität geeignet ist.

### Herstellung der erfindungsgemäßen Verbindungen

Für die Herstellung der erfindungsgemäßen Verbindungen der allgemeinen Formel I werden vor allem zwei generell anwendbare Synthesestrategien verwendet.
Einerseits lassen sich insbesondere 3,16-geschützte Abkömmlinge der Estra-1,3,5(10)-trien-3,16ξ-diole, gegebenenfalls aber auch die freien Diole, für Modifikationen einzelner Positionen des Steroidgerüstes einsetzen.
Andererseits bieten entsprechend modifizierte Estronanaloga, die in großer Zahl auf bekannten Wegen [exemplarische Syntheseverfahren siehe J. Chem. Soc. Perk. 1, 1973, 2095 für C(9); Steroids 54, 1989, 71 für C(7)] erhalten werden können, durch Transposition der Sauerstofffunktionalität (Z. Chem. 1970, 221) von C(17) nach C(16) einen flexiblen Zugang zu den erfindungsgemäßen Verbindungen.
Für den Fall des 3-Methylethers erfolgt nach Überführung des Ketons in ein Sulfonylhydrazon, im einfachsten Falle durch Umsetzung mit Phenylsulfonylhydrazid, in einer Abbaureaktion die Bildung des C(16)-C(17)Olefins (Z. Chem. 1970, 10, 221ff; Liebigs Ann. Chem. 1981, 1973ff), an das in regio/ stereokontrollierter Weise Hypobromid angelagert wird. Reduktive Dehalogenierung und Entfernung der Schutzgruppe an C(3) ergeben den 16β-Alkohol, der nach bekannten Methoden in das 16α-Epimer überführt werden kann.
Eine weitere Variante für die Einführung der Hydroxylgruppe an C-Atom 16 besteht in der Hydroborierung der 16(17)-Doppelbindung mit sterisch anspruchsvollen Boranen. Von dieser Reaktion ist bekannt, dass sie zu 16-oxygenierten Produkten führt (Indian J. Chem. 1971, 9, 287-8). Dementsprechend ergibt die Umsetzung der Estra-1,3,5(10),16-tetraene mit beispielsweise 9-Borabicyclo[3.3.1]nonan nach der Oxidation mit alkalischem Wasserstoffperoxid 16α-Hydroxyestratriene. In untergeordnetem Maße werden bei dieser Reaktion die epimeren 16β-Hydroxysteroide gebildet. Nach der Spaltung der 3-Methoxygruppe werden Estra-1,3,5(10)-trien-3,16α-diole erhalten. Durch Inversion der Konfiguration an C-Atom 16, z.B. durch Mitsunobu-Reaktion (Synthesis 1980,1) werden wiederum die 16β-Hydroxyestratriene erhalten.
Für weitere Herstellungsmöglichkeiten der C(16)-C(17) olefinischen Zwischenstufe siehe auch DE 199 06159 A1.

Die Einführung der Fluorsubstituenten erfolgt über nukleophile Substitutionsreaktionen von Hydroxylgruppen mit Fluoraminreagenzien (Org. React. 1974, 21, 158-173). Werden die Hydroxylgruppen zuvor in die entsprechenden Tosylate überführt, dann werden die fluorierten Verbindungen durch Umsetzung mit Tetra-n-butylammoniumfluorid erhalten (J. Chem. Res. (M) 1979, S. 4728-4755). Ebenfalls zugänglich sind Fluorverbindungen durch Reaktion der entsprechenden Alkohole mit Diethylaminoschwefeltrifluorid (DAST) (US 3 976 691). Geminale Difluorverbindungen werden beispielsweise durch Reaktion von Carbonylverbindungen mit Schwefeltetratfluorid (US 3 413 321) oder Diethylaminoschwefeltrifluorid (DAST) hergestellt (US 3 979 691).
Zur Synthese der erfindungsgemäßen 9α-substituierten 17β-Fluorestra-1,3,5(10)-trien-3,16-diole werden 17-Oxo-estra1,3,5(10)-triene in die 17,17-Difluorestra-1,3,5(10)-triene überführt (US 3 976 691). Die so zugänglichen 17,17-Difluorestra-1,3,5(10)-triene werden durch Behandlung mit Aluminiumoxid in 17-Fluorestra-1,3,5(10),16-tetraene umgewandelt (US 3 413 321). Eine weitere Möglichkeit zur Herstellung der Fluorolefine besteht in der Umsetzung der entsprechenden Ketone mit Diethylaminoschwefeltrifluorid (DAST) in Gegenwart polarer Katalysatoren, wie rauchender Schwefelsäure (US 4 212 815). Die Umsetzung der 17-Fluorestra-1,3,5(10),16-tetraene mit Boranen und anschließende Oxidation mit alkalischem Wasserstoffperoxid liefert die 17β-Fluorestra-1,3,5(10)-trien-16α-ole (Org. React. 1963, 13, 1-54).

Der Zugang zu den erfindungsgemäßen 9α-Alkenyl- bzw. 9α-Alkinylsubstituierten Estra-1,3,5(10)-trien-3,16α-diolen erfolgt zunächst aus den in 3- und 16-Stellung geschützten 3,16-Dihydroxy-estra-1,3,5(10)-trienen. Durch Umsetzung mit Trimethylsilylcyanid in Anwesenheit von Lithiumperchlorat erfolgt die regio- und stereoselektive Einführung einer 9α-Cyanogruppierung (Synlett, 1992, 821-2). Nach Abspaltung der Schutzgruppen wird die 9α-Cyanoverbindung durch Reduktion zunächst in eine 9α-Formylverbindung und diese anschließend durch eine Wittig-Reaktion (Org. React. Vol.14, 270) in die 9α-Alkenyl- bzw. 9α-Alkinyl-substituierten Verbindung überführt.

Die erfindungsgemäßen Estratrien-Sulfamate sind in an sich bekannter Weise aus den entsprechenden Hydroxy-Steroiden durch Veresterung mit Sulfamoylchloriden in Gegenwart einer Base zugänglich [Z. Chem. **15**, 270-272 (1975); Steroids **61**, 710 - 717 (1996)].
Nachfolgende Acylierung der Sulfamatgruppe führt zu den erfindungsgemäßen (N-Acyl)sulfamaten. Für die (N-Acyl)sulfamate wurden bereits pharmakokinetische Vorteile nachgewiesen (vgl. DE 195 40 233 A1).

Die regioselektive Veresterung von polyhydroxylierten Steroiden mit N-substituierten und N-unsubstituierten Sulfamoylchloriden erfolgt nach partiellem Schutz derjenigen Hydroxylgruppen, die unverestert bleiben sollen. Als Schutzgruppen mit hierfür geeigneter selektiver Reaktivität haben sich Silylether ervviesen, da diese unter den Bedingungen der Sulfamatbildung stabil sind und die Sulfamatgruppe intakt bleibt, wenn der/ die Silylether zur Regenerierung der (restlichen) im Molekül noch enthaltenen Hydroxylgruppe(n) wieder abgespalten werden (Steroids **61**, 710 - 717 (1996)).
Die Herstellung der erfindungsgemäßen Sulfamate mit einer zusätzlichen Hydroxylgruppe im Molekül ist auch dadurch möglich, dass man von geeigneten Hydroxy-Steroidketonen ausgeht. Zunächst werden, je nach Zielstellung, eine oder mehrere vorhandene Hydroxylgruppen einer Sulfamoylierung unterworfen. Dann können die Sulfamatgrupen gegebenenfalls mit einem gewünschten Acylchlorid in Gegenwart einer Base in die betreffenden (N-Acyl)sulfamate überführt werden. Die nunmehr vorliegenden Oxosulfamate oder Oxo-(N-acyl)sulfamate werden durch Reduktion in die entsprechenden Hydroxysulfamate bzw. Hydroxy-(N-acyl)sulfamate umgewandelt (Steroids **61**, 710 - 717 (1996)). Als geeignete Reduktionsmittel kommen Natriumborhydrid und der Boran-Dimethylsulfid-Komplex in Frage.

Die nachfolgenden Beispiele dienen der näheren Erläuterung der Erfindung.

Analog zum Aufbau der 9α-Vinylgruppierung können unter Verwendung homologer Reagenzien zu den in den Beispielen beschriebenen Reagenzien weitere Verbindungen der allgemeinen Formel I erhalten werden.

Veretherung und/ oder Veresterung freier Hydroxygruppen erfolgen nach dem Fachmann gängigen Methoden.

### Beispiel 1

### 9α-Vinylestra-1,3,5(10)-trien-3,16α-diol

### Stufe 1

### 9α-Cyano-3-methoxy-estra-1,3,5(10)-trien-16α-yl-acetat

Zu einer Suspension aus 2,13 g (6,49 mmol) 3-Methoxy-estra-1,3,5(10)-trien-16α-yl-acetat, 2,07 ml (16,54 mmol) Trimethylsilylcyanid und 0,14 g Lithiumperchlorat in 100 ml Methylenchlorid gibt man unter Rühren tropfenweise eine Lösung von 2,21 g (9,73 mmol) 2,3-Dichlor-5,6-dicyano-1,4-benzochinon in 80 ml Methylenchlorid. Das Reaktionsgemisch färbt sich grün. Nach 1 Stunde Reaktionszeit bei Raumtemperatur wird das Gemisch mit Natriumhydrogencarbonatlösung versetzt. Die abgetrennte organische Phase wäscht man mit Wasser und engt i.V. ein. Das Produktgemisch wird an Kieselgel (Cyclohexan/Essigester, 6/1) chromatografiert. Man erhält 0,44 g (21 %) 9α-Cyano-3-methoxy-estra-1,3,5(10)-trien-16α-yl-acetat.

### Stufe 2

### 9α-Cyano-3-hydroxy-estra-1,3,5(10)-trien-16α-yl-acetat

Zu einer Lösung aus 0,59 g (1,67 mmol) 9α-Cyano-3-methoxy-estra-1,3,5(10)-trien-16α-yl-acetat in 30 ml Acetonitril gibt man unter Rühren in einer Argonatmosphäre 7,51 g (50,1 mmol) Natriumiodid und 8,87 ml (70,14 mmol) Trimethylchlorsilan. Nach ca. 3 Stunden bei 60-70°C ist die Reaktion beendet. Die Reaktionslösung wird in Natriumhydrogensulfitlösung gegeben und mit Essigester extrahiert. Die organische Phase wäscht man mehrmals mit Wasser, trocknet über MgSO₄ und engt i.V. bis zur Trockne ein.
Das Rohproduktes wird an Kieselgel (Cyclohexan/Essigester, 4/1) chromatografiert. Man erhält 0,43 g (76 %) Produkt.

### Stufe 3

### 3,16α-Dihydroxyestra-1,3,5(10)-trien-9 carbonitril

Bei Raumtemperatur werden 0,43 g (1,27 mmol) 9α-Cyano-3-hydroxy-estra-1,3,5(10)-trien-16α-yl-acetat mit 1,0 g (7,24 mmol) Kaliumkarbonat in 40 ml Methanol (1 % Wasser) 2 Stunden gerührt. Anschließend destilliert man das Methanol i.V. ab und löst den organischen Rückstand in Methylenchlorid. Die organische Phase wird mit Wasser gewaschen und eingeengt. Man erhält 3,5 g (93 %) 9α-Cyano-estra-1,3,5(10)-trien-3,16α-diol.

### Stufe 4

### 3,16α-Dihydroxyestra-1,3,5(10)-trien-9 carbaldehyd

Eine Suspension aus 100 mg (0,34 mmol) 3,16α-Dihydroxyestra-1,3,5(10)-trien-9 carbonitril in 40 ml Toluol wird unter Rühren auf ca. -20°C abgekühlt. Nach Zugabe von 0,9 ml (1,35 mmol) Diisobutylaluminiumhydrid wird die Reaktion nach ca. 10 min mit Natriumhydrogencarbonatlösung versetzt, über Celite filtriert und das Filterhilfsmittel mit Essigester nachextrahiert. Die vereinigten organischen Phasen werden mit Wasser gewaschen. Durch Einengen der Lösung i.V. erhält man 84,6 mg eines hellgelben Schaumes. Das im Gemisch enthaltene Produkt entspricht einer Ausbeute von ca. 52 % d.Th und wird ohne weitere chromatographische Aufarbeitung in der nächsten Stufe eingesetzt.

### Stufe 5

### 9α-Vinylestra-1,3,5(10)-trien-3,16α-diol

Unter Inertgasatmosphäre werden 3,1 g (7,9 mmol) Triphenylmethylphosphoniumiodid und 0,24 g (8 mmol) Natriumhydrid (80 %-ig in Paraffinöl) in 20 ml DMSO im Ultraschallbad bei ca. 55 °C zur Reaktion gebracht. Nach 10 min gibt man zur Lösung 80 mg (0,16 mmol, ca. 60 %-ig) 3,16α-Dihydroxyestra-1,3,5(10)-trien-9 carbaldehyd und läßt das Gemisch noch 60 min bei ca. 55°C im Ultraschallbad reagieren. Nach Zugabe von Wasser wird mit Essigester extrahiert. Die gesammelten organischen Phasen wäscht man mit Wasser und engt die organische Phase i. V. ein.
Das Rohprodukt wird durch Säulenchromatographie an Kieselgel; (Cyclohexan/Essigester, 2/1) und anschließende Umkristallisation aus Chloroform gereinigt. Ausbeute: 24 mg (50 %), Schmelzpunkt 88 - 95 °C.
¹H-NMR (400 MHz, DMSO-d₆, TMS): 9,00 (s, 3-OH); 6,98 (d, J = 8,6 Hz, H-1); 6,49 (dd, J = 8,6/2,7 Hz, H-2); 6,41 (d, J = 2,7 Hz, H-4); 6,25 (dd, J = 17,2/10,5 Hz, -CH=CH₂); 5,00 (dd, 10,5/1,9 Hz, -CH=CH₂); 4,47 (d, 4,69 Hz, 16α-OH); 4,45 (dd, 17,2/1,9 Hz, -CH=CH₂); 4,24 (m, 16β-H); 2,68 (m, H-6); 0,69 (s, H-18)

### Beispiel 2

### 9α-Vinyl-18a-homo-estra-1,3,5(10)-trien-3,16α-diol

### Stufe 1

### 3,16α-Bis[(perhydropyran-2-yl)oxy]-18a-homo-estra-1,3,5(10)-trien-9-carbonitril

1.03 g (2,26 mmol) 3,16α-Bis[(perhydropyran-2-yl)oxy]-18a-homo-estra-1,3,5(10)-trien, 48,2 mg (0,45 mmol) Lithiumperchlorat und 0,71 ml (5,66 mmol) Trimethylsilylcyanid werden in 10 ml Methylenchlorid (Molsieb) vorgelegt und unter Inertgas auf ca. -70°C unter Rühren abgekühlt. Anschließend tropft man 0,77 g (3,39 mmol) 2,3-Dichlor-5,6-dicyano-1,4-benzochinon gelöst in 65 ml Methylenchlorid innerhalb von 1 Stunde zu. Nach ca. 1 Stunde (Erwärmung auf Raumtemperatur) wird die Reaktionslösung mit Natriumhydrogencarbonatlösung versetzt und die Reaktionsprodukte mit Methylenchlorid extrahiert. Das durch Einengen der organischen Phasen erhaltene Rohprodukt wird chromatographisch gereinigt. Nach der Chromatographie an Kieselgel (Cyclohexan/Essigester, 4/1) erhält man 0,74 g (68 % d.Th.) Produkt.

### Stufe 2

### 3,16α-Dihydroxy-18a-homo-estra-1,3,5(10)-trien-9-carbaldehyd

1,3 g (2,7 mmol) 3,16α-Bis[(perhydropyran-2-yl)oxy]-18a-homo-estra-1,3,5(10)-trien-9-carbonitril werden in 40 ml Toluol gelöst und bei Raumtemperatur unter Inertgas mit 7,2 ml (10,8 mmol) Diisobutylaluminiumhydridlösung (1,5 M in Toluol) versetzt. Nach einer Reaktionszeit von 30 Minuten gibt man zur Reaktionslösung ein Gemisch von 30 ml Methanol und 5 ml verdünnter Salzsäure (1/1). Die Reaktionslösung wird unter Vakuum eingeengt und der Rückstand in Essigester aufgenommen. Die erhaltene organische Phase extrahiert man mit Wasser und wäscht mit Natriumhydrogencarbonatlösung.

Nach Trocknung der Lösung und Einengen unter Vakuum werden 0,73 g (86% d.Th.) gelbe Kristalle erhalten.

### Stufe 3

### 9α-Vinyl-18a-homo-estra-1,3,5(10)-trien-3,16α-diol

Unter Inertgasatmosphäre werden 13,7 g (34,8 mmol) Triphenylmethylphosphoniumjodid und 1,0 g (34,8 mmol) Natriumhydrid (ca. 80% auf Paraffinöl) in 80 ml DMSO im Ultraschallbad bei ca. 50°C zur Reaktion gebracht. Nach 30 Minuten gibt man 0,73 g (2,3 mmol) 3,16α-Dihydroxy-18a-homo-estra-1,3,5(10)-trien-9-carbaldehyd gelöst in 10 ml DMSO zur Reaktionslösung und läßt das Gemisch weitere 60 Minuten im Ultraschallbad reagieren. Nach Zugabe von Wasser wird mit Essigester extrahiert, die organische Phase mit Wasser gewaschen, getrocknet und eingeengt.
Das Rohprodukt reinigt man durch Säulenchromatographie an Kieselgel (Cyclohexan/Essigester, 2/1) und Kristallisation aus Chloroform.

| | |
|---|---|
| Ausbeute | 0,59 g (81% d.Th.) nach Chromatographie |
| Schmelzpunkt | 214 - 220 °C |

¹H-NMR (400 MHz, DMSO-d₆, TMS): 9,00 (s, 3-OH); 6,96 (d, J = 8,6 Hz, H-1); 6,49 (dd, J = 8,6/2,7 Hz, H-2); 6,41 (d, J = 2,7 Hz, H-4); 6,29 (dd, J = 17,2/10,5 Hz, -CH=CH₂); 5,00 (dd, J = 10,5/1,9 Hz, -CH=CH₂); 4,48 (d, J = 4,7 Hz, 16α-OH); 4,43 (dd, J = 17,2/1,9 Hz, -CH=CH₂); 4,18 (m, 16β-H); 2,68 (m, H-6); 0,72 (t, J = 6,8 Hz, H-18a).

### Beispiel 3

### 9α-(2',2'-Difluorvinyl)-estra-1,3,5(10)-trien-3,16α-diol

### Stufe 1

### 3,16α-Bis[(perhydropyran-2-yl)oxy]-estra-1,3,5(10)-trien-9-carbonitril

Umsetzung von 3,16α-Bis[(perhydropyran-2-yl)oxy]-estra-1,3,5(10)-trien analog zum Beispiel 1, Stufe 1 ergibt 3,16α-Bis[(perhydropyran-2-yl)oxy]-estra-1,3,5(10)-trien-9-carbonitril Ausbeute: 58 % d.Th.

### Stufe 2

### 3,16α-Dihydroxy-estra-1,3,5(10)-trien-9-carbaldehyd

Umsetzung von 3,16α-Bis[(perhydropyran-2-yl)oxy]-estra-1,3,5(10)-trien-9-carbonitril analog zum Beispiel 1, Stufe 2 ergibt 3,16α-Dihydroxy-estra-1,3,5(10)-trien-9-carbaldehyd;

| | |
|---|---|
| Ausbeute | 83 % d.Th. |

### Stufe 3

### 9α-(2,2-Difluorvinyl)-estra-1,3,5(10)-trien-3,16α-diol

In einem inertisierten Reaktionskolben werden 1,5 ml Dimethoxyethan (Molsieb), 0,3 ml Pentan und 0,13 ml (0,77 mmol) Diethyl(difluormethyl)-phosphonat vorgelegt und auf ca. -75°C abgekühlt. Nach Zugabe von 0,72 ml (1,07 mmol) *tert*-Butyllithium (1,5 M in Pentan) und 30 Minuten Reaktionszeit gibt man zur Reaktionslösung 0,14 g (0,31 mmol) 3,16α-Dihydroxy-estra-1,3,5(10) trien-9-carbaldehyd gelöst in einem Gemisch von 1,5 ml Dimethoxyethan / 0,3 ml Pentan. Die Reaktionslösung wird zur vollständigen Umsetzung bis zum Rückfluß erhitzt. Nach Zugabe in gekühlte Ammoniumchloridlösung extrahiert man mit Essigester. Die organische Phase wird unter Vakuum eingengt, der Rückstand in 5 ml Methanol aufgenommen und mit 0,5 ml verdünnter Salzsäure (1 / 1) versetzt. Zur Reaktionslösung gibt man Essigester, wäscht die organische Phase mit Natriumhydrogencarbonatlösung und engt unter Vakuum ein. Das erhaltene Rohprodukt wird durch Säulenchromato-graphie an Kieselgel (Cyclohexan/Essigester, 2/1) gereinigt.

| | |
|---|---|
| Ausbeute | 22 mg (21 % d.Th.) |

¹H-NMR (400 MHz, DMSO-d₆, TMS): 9,08 (s, 3-OH); 7,10 (d, J = 8,6 Hz, H-1); 6,51 (dd, J = 8,6/2,3 Hz, H-2); 6,41 (d, J = 2,3 Hz, H-4); 4,76 (dd, J = 25,4/10,9 Hz, -CH=CF₂); 4,51 (d, J = 4,69 Hz, 16α-OH); 4,25 (m, 16β-H); 2,68 (m, H-6); 0,68 (s, H-18)

### Beispiel 4

### 9α-(2',2'-Difluorvinyl)-18a-homo-estra-1,3,5(10)-trien-3,16α-diol

### Stufe 1

### 3,16α-Bis[(perhydropyran-2-yl)oxy]-18a-homo-estra-1,3,5(10)-trien-9-carbonitril

Umsetzung von 3,16α-Bis[(perhydropyran-2-yl)oxy]-18a-homo-estra-1,3,5(10)-trien analog zum Beispiel 1, Stufe 1 ergibt 3,16α-Bis[(perhydropyran-2-yl)oxy]-18a-homoestra-1,3,5(10)-trien-9-carbonitril;
Ausbeute: 58 % d.Th. ;

### Stufe 2

### 3,16α-Dihydroxy-18a-homo-estra-1,3,5(10)-trien-9-carbaldehyd

Umsetzung von 3,16α-Bis[(perhydropyran-2-yl)oxy]-18a-homo-estra-1,3,5(10)-trien-9-carbonitril analog zum Beispiel 1, Stufe 2 ergibt 3,16α-Dihydroxy-18a-homo-estra-1,3,5(10)-trien-9-carbaldehyd;

| | |
|---|---|
| Ausbeute | 87 % d.Th |

### Stufe 3

### 9α-(2,2-Difluorvinyl)-18a-homo-estra-1,3,5(10)-trien-3,16α-diol

Umsetzung von 3,16α-Dihydroxy-18a-homo-estra-1,3,5(10)-trien-9-carbaldehyd; Reaktionsbedingungen und -durchführung sowie Molverhältnisse wie bei 3. Stufe 9α-(2,2-Difluorvinyl)-estra-1,3,5(10)-trien-3,16α-diol
Das Rohprodukt wird durch Säulenchromatographie an Kieselgel (Cyclohexan / Essigester, 2 / 1) und Kristallisation aus Essigester gereinigt.

| | |
|---|---|
| Ausbeute | 12 % d.Th. |
| Schmelzpunkt | 225 - 232 °C |

¹H-NMR (400 MHz, DMSO-d₆, TMS): 9,06 (s, 3-OH); 7,08 (d, J = 8,6 Hz, H-1); 6,50 (dd, J = 8,6/2,7 Hz, H-2); 6,41 (d, J = 2,7 Hz, H-4); 4,78 (dd, J = 21,5/14,8 Hz, -CH=CF₂); 4,47 (d, J = 4,50 Hz, 16α-OH); 4,18 (m, 16β-H); 2,68 (m, H-6); 0,72 (t, J = 6,8 Hz, H-18a)

### Beispiel 5

### 17β-Fluor-9α-vinyl-estra-1,3,5(10)-trien-3,16α-diol

### Stufe 1

### 3,16α-Bis[(perhydropyran-2-yl)oxy]-17β-fluor-estra-1,3,5(10)-trien-9-carbonitril

Umsetzung von 3,16α-Bis[(perhydropyran-2-yl)oxy]-17β-fluor-estra-1,3,5(10)-trien analog zum Beispiel 2, Stufe 1 ergibt 3,16α-Bis[(perhydropyran-2-yl)oxy]-17β-fluor-estra-1,3,5(10)-trien-9-carbonitril;

| | |
|---|---|
| Ausbeute | 45 % d.Th. |

### Stufe 2

### 3,16α-Dihydroxy-17β-fluor-estra-1,3,5(10)-trien-9-carbaldehyd

Umsetzung von 3,16α-Bis[(perhydropyran-2-yl)oxy]- 17β-fluor-estra-1,3,5(10)-trien-9-carbonitril analog zum Beispiel 2, Stufe 2 ergibt 3,16α-Dihydroxy-17β-fluor-estra-1,3,5(10)-trien-9-carbaldehyd;

| | |
|---|---|
| Ausbeute | 83 % d.Th. |

### Stufe 3

### 17β-Fluor-9α-vinyl-estra-1,3,5(10)-trien-3,16α-diol

Umsetzung von 3,16α-Dihydroxy-17β-fluor-estra-1,3,5(10)-trien-9-carbaldehyd analog zum Beispiel 2, Stufe 3 ergibt 17β-Fluor-9α-vinyl-estra-1,3,5(10)-trien-3,16α-diol;
Das Rohprodukt wird durch Säulenchromatographie an Kieselgel (Cyclohexan / Essigester, 2 / 1) und Kristallisation aus Chloroform gereinigt.

| | |
|---|---|
| Ausbeute | 51 % d.Th. |
| Schmelzpunkt | 94 - 98°C |

¹H-NMR (400 MHz, DMSO-d₆, TMS): 9,02 (s, 3-OH); 6,97 (d, J = 8,2 Hz, H-1); 6,51 (dd, J = 8,2/2,7 Hz, H-2); 6,42 (d, J = 2,7 Hz, H-4); 6,22 (dd, J = 17,2/10,5 Hz, -CH=CH₂); 5,09 (d, J = 5,5 Hz, 16α-OH); 5,01 (dd, J = 10,5/1,9 Hz, -CH=CH₂); 4,45 (dd, J =17,2/1,9 Hz, -CH=CH₂); 4,35 (dd, J = 55,1/4,7 Hz, H-17α); 4,11 (m, 16β-H); 2,68 (m, H-6); 0,79 (d, J = 1,9 Hz, H-18)

### Beispiel 6

### 17,17-Difluor-9α-vinyl-estra-1,3,5(10)-trien-3,16α-diol

### Stufe 1

### 3,16α-Bis[(perhydropyran-2-yl)oxy]-17,17-difluor-estra-1,3,5(10)-trien-9-carbonitril

Umsetzung von 3,16α-Bis[(perhydropyran-2-yl)oxy]-17,17-difluor-estra-1,3,5(10)-trien analog zum Beispiel 2, Stufe 1 ergibt 3,16α-Bis[(perhydropyran-2-yl)oxy]-17,17-difluor-estra-1,3,5(10)-trien-9-carbonitril;

| | |
|---|---|
| Ausbeute | 46 % d.Th. |

### Stufe 2

### 3,16α-Dihydroxy-17,17-difluor-estra-1,3,5(10)-trien-9-carbaldehyd

Umsetzung von 3,16α-Bis[(perhydropyran-2-yl)oxy]-17,17-difluor-estra-1,3,5(10)-trien-9-carbonitril analog zum Beispiel 2, Stufe 2 ergibt 3,16α-Dihydroxy-17,17-difluor-estra-1,3,5(10)-trien-9-carbaldehyd;

| | |
|---|---|
| Ausbeute | 88 % d.Th. |

### Stufe 3

### 17,17-Difluor-9α-vinyl-estra-1,3,5(10)-trien-3,16α-diol

Umsetzung von 3,16α-Dihydroxy-17,17-difluor-estra-1,3,5(10)-trien-9-carbaldehyd analog zum Beispiel 2, Stufe 3 ergibt 17,17-Difluor-9α-vinyl-estra-1,3,5(10)-trien-3,16α-diol;

Das Rohprodukt wird durch Säulenchromatographie an Kieselgel (Cyclohexan / Essigester, 2/1) und Kristallisation aus Chloroform gereinigt.

| | |
|---|---|
| Ausbeute | 75 % d.Th. |

¹H-NMR (400 MHz, CDCl₃, TMS): 7,08 (d, J = 8,6 Hz, H-1); 6,63 (dd, J = 8,6/2,7 Hz, H-2); 6,54 (d, J = 2,7 Hz, H-4); 6,23 (dd, J = 17,2/10,5 Hz, -CH=CH₂); 5,08 (dd, J = 10,5/1,9 Hz, -CH=CH₂); 4,48 (dd, J = 17,2/1,9 Hz, -CH=CH₂); 4,44 (m, 16β-H); 2,79 (m, H-6); 0,95 (d, J = 1,9 Hz, H-18)

### Beispiel 7

### 9α-(Hex-1'-enyl)-estra-1,3,5(10)-trien-3,16α-diol

### Stufe 1

### 3,16α-Bis[(perhydropyran-2-yl)oxy]-estra-1,3,5(10)-trien-9-carbonitril

Umsetzung von 3,16α-Bis[(perhydropyran-2-yl)oxy]-estra-1,3,5(10)-trien analog zum Beispiel 2, Stufe 1 ergibt 3,16α-Bis[(perhydropyran-2-yl)oxy]-estra-1,3,5(10)-trien-9-carbonitril;

| | |
|---|---|
| Ausbeute | 61 % d.Th. |

### Stufe 2

### 3,16α-Dihydroxy-estra-1,3,5(10)-trien-9-carbaldehyd

Umsetzung von 3,16α-Bis[(perhydropyran-2-yl)oxy]-estra-1,3,5(10)-trien-9-carbonitril analog zum Beispiel 2, Stufe 2 ergibt 3,16α-Dihydroxy-estra-1,3,5(10)-trien-9-carbaldehyd;

| | |
|---|---|
| Ausbeute | 87 % d.Th. |

### Stufe 3

### 9α-(Hex-1-enyl)-estra-1,3,5(10)-trien-3,16α-diol

In einem inertisierten Reaktionskolben werden 8,68 g (20 mmol) Pentyltriphenylphosphoniumbromid + Natriumamid (1g enthalten 2,3 mmol Pentyltriphenylphosphoniumbromid), 0,2 g (0,67 mmol) 3,16α-Dihydroxy-estra-1,3,5(10)-trien-9-carbaldehyd und 30 ml DMSO vorgelegt. Die Reaktionsmischung wird ca. 2 Stunden im Ultraschallbad bei 60°C behandelt. Nach vollständiger Umsetzung gibt man Wasser zur Reaktionslösung. Isoliert wird das Rohprodukt durch Extraktion mit Essigester, Waschen der organischen Phase mit Wasser und Einengen bis zur Trockne.
Das erhaltene Rohprodukt wird durch Säulenchromatographie an Kieselgel (Cyclohexan / Essigester, 1/1) und Kristallisation aus Essigester gereinigt.

| | |
|---|---|
| Ausbeute | 0,18 g (75 % d.Th.) nach Chromatographie |
| Schmelzpunkt | 166 -168 °C |

¹H-NMR (400 MHz, DMSO-d₆, TMS): 8,97 (s, 3-OH); 7,08 (d, J = 8,6 Hz, H-1); 6,49 (dd, J = 8,6/2,7 Hz, H-2); 6,41 (d, J = 2,7 Hz, H-4); 5,73 (d, J = 12,5 Hz, -CH=CH-CH₂-); 5,20 (dt, J = 12,5/7,4 Hz, -CH=CH-CH₂-); 4,48 (d, J = 4,7 Hz, 16α-OH); 4,24 (m, 16β-H); 2,66 (m, H-6); 0,68 (t, J = 7,0 Hz, CH₃-CH₂-); 0,66 (s, H-18)

### Beispiel 8

### 9α-(But-1'-enyl)-estra-1,3,5(10)-trien-3,16α-diol

### Stufe 1

### 3,16α-Bis[(perhydropyran-2-yl)oxy]-estra-1,3,5(10)-trien-9-carbonitril

Umsetzung von 3,16α-Bis[(perhydropyran-2-yl)oxy]-estra-1,3,5(10)-trien analog zum Beispiel 2, Stufe 1 ergibt 3,16α-Bis[(perhydropyran-2-yl)oxy]-estra-1,3,5(10)-trien-9-carbonitril;

| | |
|---|---|
| Ausbeute | 52 % d.Th. |

### Stufe 2

### 3,16α-Dihydroxy-estra-1,3,5(10)-trien-9-carbaldehyd

Umsetzung von 3,16α-Bis[(perhydropyran-2-yl)oxy]-estra-1,3,5(10)-trien-9-carbonitril analog zum Beispiel 2, Stufe 2 ergibt 3,16α-Dihydroxy-estra-1,3,5(10)-trien-9-carbaldehyd;

| | |
|---|---|
| Ausbeute | 87 % d.Th. |

### Stufe 3

### 9α-(But-1-enyl)-estra-1,3,5(10)-trien-3,16α-diol

In einem inertisierten Reaktionskolben werden 8,68 g (20 mmol) Propyltriphenylphosphoniumbromid + Natriumamid (1g enthalten 2,3 mmol Propyltriphenylphosphoniumbromid), 0,2 g (0,67 mmol) 3,16α-Dihydroxy-estra-1,3,5(10)-trien-9-carbaldehyd und 30 ml DMSO vorgelegt. Die Reaktionsmischung wird ca. 2 Stunden im Ultraschallbad bei 60°C behandelt. Nach vollständiger Umsetzung gibt man Wasser zur Reaktionslösung. Isoliert wird das Rohprodukt durch Extraktion mit Essigester, Waschen der organischen Phase mit Wasser und Einengen bis zur Trockne.
Das erhaltene Rohprodukt wird durch Säulenchromatographie an Kieselgel (Cyclohexan / Essigester, 1/1) gereinigt.

| | |
|---|---|
| Ausbeute | 0,16 g (73 % d.Th.) nach Chromatographie |
| Schmelzpunkt | 140 -148 °C |

¹H-NMR (400 MHz, DMSO-d₆, TMS): 8,98 (s, 3-OH); 7,09 (d, J = 8,6 Hz, H-1); 6,49 (dd, J = 8,6/2,7 Hz, H-2); 6,41 (d, J = 2,7 Hz, H-4); 5,70 (d, J = 12,5 Hz, -CH=CH-CH₂-); 5,19 (dt, J = 12,5/7,4 Hz, -CH=CH-CH₂-); 4,47 (d, J = 4,7 Hz, 16α-OH); 4,24 (m, 16β-H); 2,66 (m, H-6); 0,66 (s, H-18); 0,57 (t, J = 7,2 Hz, CH₃-CH₂-)

## Patentansprüche

1. 9a-substituierte Estra-1,3, 5(10)-trienderivate der allgemeinen Formel (I) 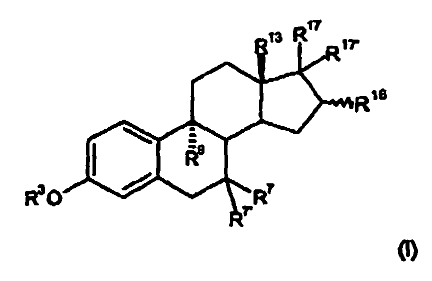 worin die Reste R³,R⁷,R⁷',R⁹,R¹³,R¹⁶ sowie R¹⁷ und R^{17'} unabhängig voneinander folgende Bedeutung besitzen:
R³ ein Wasserstoffatom oder eine Gruppe R¹⁸, worin
R¹⁸ ein gerad-oder verzweigtkettiger, gesättigter oder ungesättigter Kohlenwasserstoffrest mit bis zu 6 Kohlenstoffatomen, eine Trifluormethylgruppe, ein Aryl-. Heteroaryl- oder Aralkylrest, ein substituierter Aryl-, Heteroarylrest mit ein Methyl-, Ethyl-, Trifluormethyl-, Pentafluorethyl-, Trifluormethylthio-, Methoxy-, Ethoxy-, Nitro-, Cyano-, Halogen- (Fluor, Chlor, Brom, lod), Hydroxy-, Amino-, Mono(C₁₋₈-alkyl)- oder Di(C₁₋₈-alkyl) amino, wobei beide Alkylgruppen identisch oder verschieden sind, Di (aralkyl) amino, wobei beide Aralkylgruppen identisch oder verschieden sind, Carboxyl, Carboxyalkoxy, C₁-C₂₀-Acyl oder C₁-C₂₀-Acyloxy-Gruppen als Substituenten, ein Acylrest COR¹⁹, worin R¹⁹ ein, gerad-oder verzweigtkettiger, gesättigter oder bis zu dreifach ungesättigter, teilweise oder vollständig halogenierter Kohlenwasser- stoffrest mit bis zu 10 Kohlenstoffatomen ist, oder
R¹⁸ eine Gruppe R²⁰SO₂-bedeutet, worin
R²⁰ eine R²¹R²²N - Gruppe ist, wobei R²¹ und R²² unabhängig voneinander ein Wasserstoffatom, einen C₁-C₆- Alkylrest, eine Gruppe C(O)R²³, worin R²³ einen gerad- oder verzweigtkettigen, gesättigten oder bis zu dreifach ungesättigten, gegbenenfalls teilweise oder vollständig halogenierten Kohlenwasserstoffrest mit bis zu 10 Kohlenstoffatomen, einen Cyclopropyl-, -butyl-,-pentyl-, -hexyl- oder -heptylgruppe, einen C₄-C₁₆-Cycloalkyl-alkylrest mit einem 3 bis 7 Kohlenstoffatome im Cycloalkylteil und mit einem Alkylteil von bis zu 8 Kohlenstoffatomen oder einen Aryl-, Heteroaryl- oder Aralkylrest darstellt, oder ein substituierter Aryl-, Heteroarylrest mit ein Methyl-, Ethyl-, Trifluormethyl-, Pentafluorethyl-, Trifluormethylthio-, Methoxy-, Ethoxy-, Nitro-, Cyano-, Halogen- (Fluor, Chlor, Brom, Iod), Hydroxy-, Amino-, Mono(C₁₋₈-alkyl)- oder Di(C₁₋₈-alkyl) amino, wobei beide Alkylgruppen identisch oder verschieden sind, Di (aralkyl) amino, wobei beide Aralkylgruppen identisch oder verschieden sind, Carboxyl, Carboxyalkoxy, C₁-C₂₀-Acyl oder C₁-C₂₀-Acyloxy-Gruppen als Substituenten oder, zusammen mit dem N-Atom, einen Polymethyleniminorest mit 4 bis 6 C-Atomen oder einen Morpholinorest, bedeuten,
R⁷ und R^{7'} jeweils unabhängig voneinander ein Wasserstoffatom oder ein Halogenatom,
R⁹ ein gerad-oder verzweigtkettiger Alkenyl-oder Alkinylrest mit 2 bis 6 Kohlenstoffatomen, der teilweise oder vollständig fluoriert sein kann, oder ein Ethinyl-oder Prop-1-inylrest
R¹³ eine Methylgruppe oder eine Ethylgruppe,
R¹⁶ eine Hydroxygruppe oder eine Gruppe R¹⁸O-, R²⁰SO₂- oder OC(O)R²³ mit R¹⁸, R²⁰ und R²³ jeweils in der unter R³ angegebenen Bedeutung,
R¹⁷ und R^{17'} jeweils unabhängig voneinander ein Wasserstoffatom oder ein Halogenatom.

2. Verbindungen der allgemeinen Formel I nach Anspruch 1, worin R³ ein Wasserstoffatom ist.

3. Verbindungen der allgemeinen Formel I nach Anspruch 1 oder 2, worin R⁷ ein Wasserstoffatom oder ein α-ständiges Fluoratom, R⁸ eine Vinyl-, Ethinyl- oder Prop-1-inylgruppe, R¹⁸ eine Hydroxygruppe und R¹⁷ ein Wasserstoffatom oder ein α-ständiges Fluoratom ist.

4. Verbindungen der allgemeinen Formel I nach Anspruch 1 oder 2, worin R¹⁶ für eine Gruppe R¹⁸-O- oder R¹⁹SO₂-O- mit R¹⁸ und R¹⁹ jeweils in der unter R³ angegebenen Bedeutung steht.

5. Estratriene der allgemeinen Formel I nach Anspruch 1 oder 2, nämlich:
9α-Vinyl-estra-1,3,5(10)-trien-3,16α-diol
9α-Allyl-estra-1,3,5(10)-trien-3,16α-diol
18a-Homo-9α-vinyl-estra-1,3,5(10)-trien-3,16α-diol
18a-Homo-9α-allyl-estra-1,3,5(10)-trien-3,16α-diol
3-Methoxy-9α-vinyl-estra-1,3,5(10)-trien-16α-ol
9α-Allyl-3-methoxy-estra-1,3,5(10)-trien-16α-ol
18a-Homo-3-methoxy-9α-vinyl-estra-1,3,5(10)-trien-16α-ol
18a-Homo-9α-allyl-3-methoxy-estra-1,3,5(10)-trien-16α-ol
9α-(2',2'-Difluorvinyl)-estra-1,3,5(10)-trien-3,16α-diol
9α-(2',2'-Difluorvinyl)-3-methoxy-estra-1,3,5(10)-trien-16α-ol
16α-Hydroxy-9α-vinyl-estra-1,3,5(10)-trien-3yl-sulfamat
9α-Allyl-16α-hydroxy-estra-1,3,5(10)-trien-3yl-sulfamat
18a-Homo-16α-hydroxy-9α-vinyl-estra-1,3,5(10)-trien-3yl-sulfamat
18a-Homo-9α-allyl-16α-hydroxy-estra-1,3,5(10)-trien-3yl-sulfamat
9α-Vinyl-estra-1,3,5(10)-trien-3,16α-diyl-disulfamat
9α-Allyl-estra-1,3,5(10)-trien-3,16α-diyl-disulfamat
18a-Homo-9α-vinyl-estra-1,3,5(10)-trien-3,16α-diyl-disulfamat
18a-Homo-9α-allyl-estra-1,3,5(10)-trien-3,16α-diyl-disulfamat
16α-Hydroxy-9α-vinyl-estra-1,3,5(10)-trien-3yl-(N-acetyl)-sulfamat
9α-Allyl-16α-hydroxy-estra-1,3,5(10)-trien-3yl-(N-acetyl)-sulfamat
18a-Homo-16α-hydroxy-9α-vinyl-estra-1,3,5(10)-trien-3yl-(N-acetyl)-sulfamat
18a-Homo-9α-allyl-16α-hydroxy-estra-1,3,5(10)-trien-3yl-(N-acetyl)-sulfamat
9α-(Prop-(Z)-enyl)-estra-1,3,5(10)-trien-3,16α-diol
9α-(n-Propyl)-estra-1,3,5(10)-trien-3,16α-diol
9α-Ethinyl-estra-1,3,5(10)-trien-3,16α-diol
9α-Vinyl-estra-1,3,5(10)-trien-3,16α-diol-diacetat
18a-Homo-9α-vinyl-estra-1,3,5(10)-trien-3,16α-diol-diacetat
16α-Valeroyloxy-9α-vinyl-estra-1,3,5(10)-trien-3-ol
16α-Acetoxy-9α-vinyl-estra-1,3,5(10)-trien-3-ol
18a-Homo-16α-acetoxy-9α-vinyl-estra-1,3,5(10)-trien-3-ol
7α-Fluor-9α-vinyl-estra-1,3,5(10)-trien-3,16α-diol
7α-Fluor-9α-allyl-estra-1,3,5(10)-trien-3,16α-diol
17β-Fluor-9α-vinyl-estra-1,3,5(10)-trien-3,16α-diol
17β-Fluor-9α-allyl-estra-1,3,5(10)-trien-3,16α-diol
18a-Homo-7α-fluor-9α-vinyl-estra-1,3,5(10)-trien-3,16α-diol
18a-Homo-7α-fluor-9α-allyl-estra-1,3,5(10)-trien-3,16α-diol
18a-Homo-17β-fluor-9α-vinyl-estra-1,3,5(10)-trien-3,16α-diol
18a-Homo-17β-fluor-9α-allyl-estra-1,3,5(10)-trien-3,16α-diol

6. Pharmazeutische Zusammensetzungen, enthaltend mindestens eine Verbindung gemäß einem der Ansprüche 1 bis 5 sowie einen pharmazeutisch verträglichen Träger.

7. Verwendung der Verbindungen der allgemeinen Formel I gemäß der voranstehenden Ansprüche 1-5 zur Herstellung von Arzneimitteln.

8. Verwendung gemäß Anspruch 7 zur Behandlung estrogendefizienzbedingter Krankheiten und Zustände bei der Frau und beim Mann.

9. Verwendung nach Anspruch 7 zur Behandlung von peri- und postmenopausalen Beschwerden.

10. Verwendung nach Anspruch 7 für die in vitro Behandlung der weiblichen Infertilität.

11. Verwendung nach Anspruch 7 für die in vivo Behandlung der weiblichen Infertilität.

12. Verwendung nach Anspruch 7 für die Therapie von hormondefizienz bedingten Beschwerden bei chirurgisch, medikamentös oder anders bedingter ovarieller Dysfunktion.

13. Verwendung nach Anspruch 7 für die Hormonersatz-Therapie (HRT).

14. Verwendung nach Anspruch 13 in Kombination mit Selektiven Estrogenrezeptormodulatoren (SERM), beispielsweise Raloxifen.

15. Verwendung nach Anspruch 7 zur Prophylaxe und Therapie eines hormondefizienzbedingten Knochenmasseverlustes.

16. Verwendung nach Anspruch 7 zur Prophylaxe und Therapie der Osteoporose.

17. Verwendung nach Anspruch 7 zur Prophylaxe und Therapie von Herzkreislauferkrankungen.

18. Verwendung nach Anspruch 7 zur Prävention und Behandlung der Prostatahyperplasie.

19. Verwendung nach Anspruch 18 in Kombination mit Antiestrogenen und selektiven Estrogenrezeptormodulatoren (SERM) zur Prophylaxe und Therapie der Prostatahyperplasie.

20. Verwendung nach Anspruch 7 zur Behandlung von Erkrankungen des Immunsystems.

21. Verwendung nach Anspruch 20 zur Behandlung von Autoimmunerkrankungen.

22. Verwendung nach Anspruch 21 zur Behandlung der Rheumatoiden Arthritis.

23. Verwendung nach Anspruch 21 zur Behandlung der Multiplen Sklerose.

## Claims

1. 9α-Substituted oestra-1,3,5(10)-triene derivatives of general formula (I) 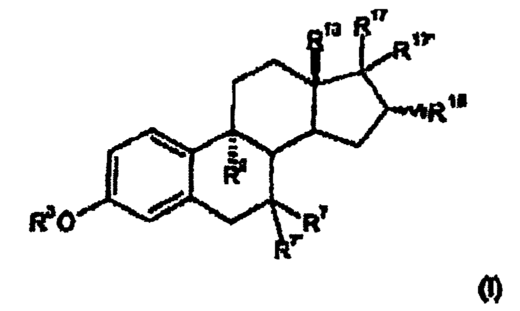 in which radicals R³, R⁷, R⁷', R⁹, R¹³, R¹⁶ as well as R¹⁷ and R^{17'}, independently of one another, have the following meaning:
R³ is a hydrogen atom or a group R¹⁸, in which
R¹⁸ is a straight-chain or branched-chain, saturated or unsaturated hydrocarbon radical with up to 6 carbon atoms, a trifluoromethyl group, an aryl, heteroaryl or aralkyl radical, a substituted aryl, heteraryl radical with a methyl, ethyl, trifluoromethyl, pentafluoroethyl, trifluoromethylthio, methoxy, ethoxy, nitro, cyano, halogen (fluorine, chlorine, bromine, iodine), hydroxyl, amino, mono(C₁₋ ₈-alkyl) or di(C₁₋₈-alkyl) amino, where both alkyl groups are identical or different, di(aralkyl)amino, where both aralkyl groups are identical or different, carboxyl, carboxyalkoxy, C₁-C₂₀-acyl or C₁-C₂₀-acyloxy groups as substituents, an acyl radical COR¹⁹, in which R¹⁹ is a straight-chain or branched-chain hydrocarbon radical with up to 10 carbon atoms that is saturated or unsaturated in up to three places and partially or completely halogenated, or
R¹⁸ is a group R²⁰SO₂, in which
R²⁰ is an R²¹R²²N group, where R²¹ and R²², independently of one another, are a hydrogen atom, a C₁-C₅-alkyl radical, a group C(O)R²³, in which R²³ is a straight-chain or branched-chain hydrocarbon radical with up to 10 carbon atoms that is saturated or unsaturated in up to three places and is partially or completely halogenated, a cyclopropyl, cyclobutyl, cyclopentyl, cyclohexyl or cycloheptyl group, a C₄-C₁₅- cycloalkylalkyl radical with 3 to 7 carbon atoms in the cycloalkyl portion and up to 8 carbon atoms in the alkyl portion, or an aryl, heteroaryl or aralkyl radical, or a substituted aryl or heteroaryl radical with a methyl, ethyl, trifluoromethyl, pentafluoroethyl, trifluoromethylthio, methoxy, ethoxy, nitro, cyano, halogen (fluorine, chlorine, bromine, iodine), hydroxyl, amino, mono(C₁₋₈-alkyl) or di(C₁₋₈-alkyl)-amino, where both alkyl groups are identical or different, di(aralkyl)-amino, whereby both aralkyl groups are identical or different, carboxyl, carboxyalkoxy, C₁-C₂₀-acyl or C₁-C₂₀-acyloxy groups as substituents or, together with the N atom, a polymethylenimino radical with 4 to 6 C atoms or a morpholino radical,
R⁷ and R^{7'}, in each case independently of one another, are a hydrogen atom or a halogen atom,
R⁹ is a straight-chain or branched-chain alkenyl or alkinyl radical with 2 to 6 carbon atoms, which can be partially or completely fluorinated, or an ethinyl or prop-1-inyl radical,
R¹³ is a methyl group or an ethyl group,
R¹⁶ is a hydroxy group or a group R¹⁸O-, R²⁰SO₂- or OC(O)R²³ with R¹⁸, R²⁰ and R²³ in each case in the meaning that is indicated under R³,
R¹⁷ and R^{17'}, in each case independently of one another, are a hydrogen atom or a halogen atom.

2. Compounds of general formula I according to Claim 1, in which R³ is a hydrogen atom.

3. Compounds of general formula I according to Claim 1 or 2, in which R⁷ is a hydrogen atom or an α-position fluorine atom, R⁹ is a vinyl, ethinyl or prop-1-inyl group, R¹⁶ is a hydroxy group, and R¹⁷ is a hydrogen atom or an α-position fluorine atom.

4. Compounds of general formula I according to Claim 1 or 2, in which R¹⁶ stands for a group R¹⁸-O- or R¹⁹SO₂-O- with R¹⁸ and R¹⁹ in each case in the meaning that is indicated under R³.

5. Oestratrienes of general formula I according to Claim 1 or 2, namely:
9α-Vinyl-oestra-1,3,5(10)-triene-3,16α-diol
9α-Allyl-oestra-1,3,5(10)-triene-3,16α-diol
18a-Homo-9α-vinyl-oestra-1,3,5(10)-triene-3,16α-diol
18a-Homo-9α-allyl-oestra-1,3,5(10)-triene-3,16α-diol
3-Methoxy-9α-vinyl-oestra-1,3,5(10)-trien-16α-ol
9α-Allyl-3-methoxy-oestra-1,3,5(10)-trien-16α-ol
18a-Homo-3-methoxy-9α-vinyl-oestra-1,3,5(10)-trien-16α-ol
18a-Homo-9α-allyl-3-methoxy-oestra-1,3,5(10)-trien-16α-ol
9α-(2',2'-Difluorovinyl)-oestra-1,3,5(10)-triene-3,16α-diol
9α-(2',2'-Difluorovinyl)-3-methoxy-oestra-1,3,5(10)-trien-16α-ol
16α-Hydroxy-9α-vinyl-oestra-1,3,5(10)-trien-3-yl-sulphamate
9α-Allyl-16α-hydroxy-oestra-1,3,5(10)-trien-3-yl-sulphamate
18a-Homo-16a-hydroxy-9a-vinyl-oestra-1,3,5(10)-trien-3-yl-sulphamate
18a-Homo-9α-allyl-16α-hydroxy-oestra-1,3,5(10)-trien-3-yl-sulphamate 9α-Vinyl-oestra-1,3,5(10)-triene-3,16α-diyl-disulphamate
9α-Allyl-oestra-1,3,5(10)-triene-3,16α-diyl-disulphamate
18a-Homo-9α-vinyl-oestra-1,3,5(10)-triene-3,16α-diyl-disulphamate
18a-Homo-9α-allyl-oestra-1,3,5(10)-triene-3,16α-diyl-disulphamate 16α-Hydroxy-9α-vinyl-oestra-1,3,5(10)-trien-3-yl-(N-acetyl)-sulphamate
9α-Allyl-16α-hydroxy-oestra-1,3,5(10)-trien-3-yl-(N-acetyl)-sulphamate
18a-Homo-16α-hydroxy-9α-vinyl-oestra-1,3,5(10)-trien-3-yl-(N-acetyl)-sulphamate
18a-Homo-9α-allyl-16α-hydroxy-oestra-1,3,5(10)-trien-3-yl-(N-acetyl)-sulphamate
9α-(Prop-(Z)-enyl)-oestra-1,3,5(10)-triene-3,16α-diol
9α-(n-Propyl)-oestra-1,3,5(10)-triene-3,16α-diol
9α-Ethinyl-oestra-1,3,5(10)-triene-3,16α-diol
9α-Vinyl-oestra-1,3,5(10)-triene-3,16α-diol-diacetate
18a-Homo-9α-vinyl-oestra-1,3,5(10)-triene-3,16α-diol-diacetate 16α-Valeroyloxy-9α-vinyl-oestra-1,3,5(10)-trien-3-ol 16α-Acetoxy-9α-vinyl-oestra-1,3,5(10)-trien-3-ol 18a-Homo-16α-acetoxy-9α-vinyl-oestra-1,3,5(10)-trien-3-ol
7α-Fluoro-9α-vinyl-oestra-1,3,5(10)-triene-3,16α-diol 7α-Fluoro-9α-allyl-oestra-1,3,5(10)-triene-3,16α-diol
17β-Fluoro-9α-vinyl-oestra-1,3,5(10)-triene-3,16α-diol 17β-Fluoro-9α-allyl-oestra-1,3,5(10)-triene-3,16α-diol
18a-Homo-7α-fluoro-9α-vinyl-oestra-1,3,5(10)-triene-3,16α-diol
18a-Homo-7α-fluoro-96-allyl-oestra-1,3,5(10)-triene-3,16α-diol
18a-Homo-17β-fluoro-9α-vinyl-oestra-1,3,5(10)-triene-3,16α-diol
18a-Homo-17β-fluoro-9α-allyl-oestra-1,3,5(10)-triene-3,16a-diol

6. Pharmaceutical compositions that contain at least one compound according to one of Claims 1 to 5 as well as a pharmaceutically compatible vehicle.

7. Use of the compounds of general formula I according to the preceding Claims 1-5 for production of pharmaceutical agents.

8. Use according to Claim 7 for treatment of oestrogen-deficiency-related diseases and conditions in women and in men.

9. Use according to Claim 7 for treatment of peri- and postmenopausal symptoms.

10. Use according to Claim 7 for the in vitro treatment of female infertility.

11. Use according to Claim 7 for the in vivo treatment of female infertility.

12. Use according to Claim 7 for the therapy of hormone-deficiency-related symptoms in surgically, medically or otherwise induced ovarian dysfunction.

13. Use according to Claim 7 for hormone replacement therapy (HRT).

14. Use according to Claim 13 in combination with selective oestrogen receptor modulators (SERM), for example raloxifene.

15. Use according to Claim 7 for prophylaxis and therapy of a hormone-deficiency-related bone mass loss.

16. Use according to Claim 7 for prophylaxis and therapy of osteoporosis.

17. Use according to Claim 7 for prophylaxis and therapy of cardiovascular diseases.

18. Use according to Claim 7 for prevention and treatment of prostate hyperplasia.

19. Use according to Claim 18 in combination with antioestrogens and selective oestrogen receptor modulators (SERM) for prophylaxis and therapy of prostate hyperplasia.

20. Use according to Claim 7 for treatment of diseases of the immune system.

21. Use according to Claim 20 for treatment of diseases of the autoimmune system.

22. Use according to Claim 21 for treatment of rheumatoid arthritis.

23. Use according to Claim 21 for treatment of multiple sclerosis.

## Revendications

1. Dérivés d'estra-1,3,5(10)-triène substitués en position 9a, de formule générale (I) 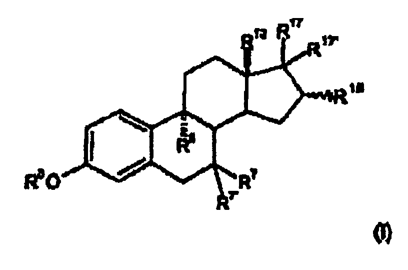 dans laquelle les radicaux R³, R⁷, R⁷', R⁹, R¹³, R¹⁶ ainsi que R¹⁷ et R¹⁷' ont, indépendamment les uns des autres, les significations suivantes :
R³ représente un atome d'hydrogène ou un groupe R¹⁸,
R¹⁸ étant un radical hydrocarboné à chaîne droite ou ramifiée, saturé ou insaturé, ayant jusqu'à 6 atomes de carbone, un groupe trifluorométhyle, un radical aryle, hétéroaryle ou aralkyle, un radical aryle, hétéroaryle substitués, comportant un groupe méthyle, éthyle, trifluorométhyle, pentafluoroéthyle, trifluorométhylthio, méthoxy, éthoxy, nitro, cyano, halogéno (fluoro, chloro, bromo, iodo), hydroxy, amino, mono [alkyl(C₁₋₈)]- ou di[alkyl(C₁₋₈)]-amino, les deux groupes alkyle étant identiques ou différents, di(aralkyl)amino, les deux groupes aralkyle étant identiques ou différents, carboxy, carboxyalkyle, acyle en C₁-C₂₀ ou acyloxy en C₁-C₂₀ en tant que substituants, un radical acyle COR¹⁹, dans lequel R¹⁹ est un radical hydrocarboné à chaîne ou ramifiée, saturé ou jusqu'à trois fois insaturé, partiellement ou totalement halogéné, ayant jusqu'à 10 atomes de carbone, ou
R¹⁸ représentant un groupe R²⁰SO₂, dans lequel
R²⁰ est un groupe R²¹R²²N, R²¹ et R²² représentant, indépendamment l'un de l'autre, un atome d'hydrogène, un radical alkyle en C₁-C₆, un groupe C(O)R²³, dans lequel R²³ représente un radical hydrocarboné à chaîne droite ou ramifiée, saturé ou jusqu'à trois fois insaturé, éventuellement partiellement ou totalement halogéné, ayant jusqu'à 10 atomes de carbone, un groupe cyclopropyle, -butyle, -pentyle, -hexyle ou -heptyle, un radical cycloalkylalkyle en C₄-C₁₅ ayant de 3 à 7 atomes de carbone dans le fragment cycloalkyle et comportant un fragment alkyle ayant jusqu'à 8 atomes de carbone, ou un radical aryle, hétéroaryle ou aralkyle, ou un radical aryle, hétéroaryle substitués comportant un groupe méthyle, éthyle, trifluorométhyle, pentafluoroéthyle, trifluorométhylthio, méthoxy, éthoxy, nitro, cyano, halogéno (fluoro, chloro, bromo, iodo), hydroxy, amino, mono[alkyl(C₁₋₈)]- ou di[alkyl(C₁₋₈)]amino, les deux groupes alkyle étant identiques ou différents, di(aralkyl)amino, les deux groupes aralkyle étant identiques ou différents, carboxy, carboxyalcoxy, acyle en C₁-C₂₀ ou acyloxy en C₁-C₂₀ en tant que substituants, ou, conjointement avec l'atome d'azote, formant ensemble un radical polyméthylène-imino ayant de 4 à 6 atomes de carbone ou un radical morpholino,
R⁷ et R^{7'} représentent chacun, indépendamment l'un de l'autre, un atome d'hydrogène ou un atome d'halogène,
R⁹ représente un radical alcényle ou alcynyle à chaîne droite ou ramifiée, ayant de 2 à 6 atomes de carbone, qui peut être partiellement ou totalement fluoré, ou un radical éthynyle ou prop-1-ynyle,
R¹³ représente un groupe méthyle ou un groupe éthyle,
R¹⁶ représente un groupe hydroxy ou un groupe R¹⁸O-, R²⁰SO₂- ou OC(O)R²³, R¹⁸, R²⁰ et R²³ ayant chacun la signification indiquée sous R³,
R¹⁷ et R^{17'} représentent, indépendamment l'un de l'autre, un atome d'hydrogène ou un atome d'halogène.

2. Composés de formule générale I selon la revendication 1, dans lesquels R³ est un atome d'hydrogène.

3. Composés de formule générale I selon la revendication 1 ou 2, dans lesquels R⁷ est un atome d'hydrogène ou un atome de fluor en positon α, R⁸ est un groupe vinyle, éthynyle ou prop-1-ynyle, R¹⁶ est un groupe hydroxy et R¹⁷ est un atome d'hydrogène ou un atome de fluor en position α.

4. Composés de formule générale I selon la revendication 1 ou 2, dans lesquels R¹⁶ représente un groupe R¹⁸-O- ou R¹⁹SO₂-O-, R¹⁸ et R¹⁹ ayant chacun la signification indiquée sous R³.

5. Estratriènes de formule générale I selon la revendication 1 ou 2, à savoir :
9α-vinyl-estra-1,3,5(10)-triène-3,16α-diol,
9α-allyl-estra-1,3,5(10)-triène-3,16α-diol,
18a-homo-9α-vinyl-estra-1,3,5(10)-triène-3,16α-diol,
18a-homo-9α-allyl-estra-1,3,5(10)-triène-3,16α-diol,
3-méthoxy-9α-vinyl-estra-1,3,5(10)-trién-16α-ol,
9α-allyl-3-méthoxy-estra-1,3,5(10)-trién-16α-ol,
18a-homo-3-méthoxy-9α-vinyl-estra-1,3,5(10)-trién-3,16α-ol,
18a-homo-9α-allyl-3-méthoxy-estra-1,3,5(10)-trién-16α-ol,
9α-(2',2'-difluorovinyl)-estra-1,3,5(10)-triène-16α-diol,
9α-(2',2'-difluorovinyl)-3-méthoxy-estra-1,3,5(10)-trién-16α-ol,
sulfamate de 16α-hydroxy-9α-vinyl-estra-1,3,5(10)-trién-3-yle,
sulfamate de 9α-allyl-16α-hydroxy-estra-1,3,5(10)-trién-3-yle,
sulfamate de 18a-homo-16α-hydroxy-9α-vinyl-estra-1,3,5(10)-trién-3-yle,
sulfamate de 18a-homo-9α-allyl-16α-hydroxy-estra-1,3,5(10)-trién-3-yle,
disulfamate de 9α-vinyl-estra-1,3,5(10)-triène-3,16α-diyle,
disulfamate de 9α-allyl-estra-1,3,5(10)-triène-3,16α-diyle,
disulfamate de 18a-homo-9α-vinyl-estra-1,3,5(10)-triène-3,16α-diyle,
disulfamate de 18a-homo-9α-allyl-estra-1,3,5(10)-triène-3,16α-diyle,
(N-acétyl)sulfamate de 16α-hydroxy-9α-vinyl-estra-1,3,5(10)-trién-3-yle,
(N-acétyl)sulfamate de 9α-allyl-16α-hydroxy-estra-1,3,5(10)-trién-3-yle,
(N-acétyl)sulfamate de 18a-homo-16α-hydroxy-9α-vinyl-estra-1,3,5(10)-trién-3-yle,
(N-acétyl)sulfamate de 18a-homo-9α-allyl-16α-hydroxy-estra-1,3,5(10)-trién-3-yle,
9α-(prop-(Z)-ényl)-estra-1,3,5(10)-triéne-3,16α-diol,
9α-(n-propyl)-estra-1,3,5(10)-triéne-3,16α-diol,
9α-éthynyl-estra-1,3,5(10)-triéne-3,16α-diol, diacétate de 9α-vinyl-estra-1,3,5(10)-triéne-3,16α-diol,
diacétate de 18a-homo-9α-vinyl-estra-1,3,5(10)-triéne-3,16α-diol,
16α-valéroyloxy-9α-vinyl-estra-1,3,5(10)-trién-3-ol,
16α-acétoxy-9α-vinyl-estra-1,3,5(10)-trién-3-ol,
18a-homo-16α-acétoxy-9α-vinyl-estra-1,3,5(10)-trién-3-ol,
7α-fluoro-9α-vinyl-estra-1,3,5(10)-triène-3,16α-diol,
7α-fluoro-9α-allyl-estra-1,3,5(10)-triène-3,16α-diol,
17β-fluoro-9α-vinyl-estra-1,3,5(10)-triène-3,16α-diol,
17β-fluoro-9α-allyl-estra-1,3,5(10)-triène-3,16α-diol,
18a-homo-7α-fluoro-9α-vinyl-estra-1,3,5(10)-triène-3,16α-diol,
18a-homo-7α-fluoro-9α-allyl-estra-1,3,5(10)-triène-3,16a-diol,
18a-homo-17β-fluoro-9α-vinyl-estra-1,3,5(10)-triène-3,16α-diol,
18a-homo-17β-fluoro-9α-allyl-estra-1,3,5(10)-triène-3,16α-diol.

6. Compositions pharmaceutiques contenant au moins un composé selon l'une quelconque des revendications 1 à 5 ainsi qu'un véhicule pharmaceutiquement acceptable.

7. Utilisation des composés de formule générale I selon les revendications précédentes 1-5, pour la fabrication de médicaments.

8. Utilisation selon la revendication 7, pour le traitement de maladies et d'états dus à une déficience en oestrogènes chez la femme et chez l'homme.

9. Utilisation selon la revendication 7, pour le traitement de troubles péri- et post-ménopausiques.

10. Utilisation selon la revendication 7, pour le traitement in vitro de l'infertilité féminine.

11. Utilisation selon la revendication 7, pour le traitement in vivo de l'infertilité féminine.

12. Utilisation selon la revendication 7, pour la thérapie de troubles dus à une déficience hormonale lors d'un dysfonctionnement ovarien d'origine chirurgicale, médicamenteuse ou autre.

13. Utilisation selon la revendication 7 pour la thérapie hormonale substitutive (HRT).

14. Utilisation selon la revendication 13, en association avec des modulateurs de récepteur d'oestrogènes sélectifs (SERM), le raloxifène par exemple.

15. Utilisation selon la revendication 7, pour la prophylaxie et la thérapie d'une perte de masse osseuse due à une déficience hormonale.

16. Utilisation selon la revendication 7, pour la prophylaxie et la thérapie de l'ostéoporose.

17. Utilisation selon la revendication 7, pour la prophylaxie et la thérapie de maladies cardiovasculaires.

18. Utilisation selon la revendication 7, pour la prévention et le traitement de l'hyperplasie de la prostate.

19. Utilisation selon la revendication 18, en association avec des anti-oestrogènes et des modulateurs de récepteur d'oestrogènes sélectifs (SERM) pour la prophylaxie et la thérapie de l'hyperplasie de la prostate.

20. Utilisation selon la revendication 7, pour le traitement de maladies du système immunitaire.

21. Utilisation selon la revendication 20, pour le traitement de maladies auto-immunes.

22. Utilisation selon la revendication 21, pour le traitement de polyarthrite rhumatoïde.

23. Utilisation selon la revendication 21, pour le traitement de la sclérose en plaques.
